# EUROPEAN PATENT SPECIFICATION

(11) **EP 2 283 129 B1**
(45) Date of publication and mention of the grant of the patent: **25.01.2017**
(21) Application number: 09753924.1
(22) Date of filing: 28.05.2009
(51) Int. Cl.: C12N 9/58, C12N 15/57, C12N 15/63, A23J 3/34

(54) **PROLINE-SPECIFIC PROTEASE FROM PENICILLIUM CHRYSOGENUM**
PROLIN-SPEZIFISCHE PROTEASE AUS PENICILLIUM CHRYSOGENUM
PROTÉASE SPÉCIFIQUES À LA PROLINE DE PENICILLIUM CHRYSOGENUM

(30) Priority: 30.05.2008 EP 08157335
(43) Date of publication of application: 16.02.2011
(73) Proprietor: DSM IP Assets B.V., 6411 TE Heerlen (NL)
(72) Inventor: DEKKER, Petrus, Jacobus, Theodorus, NL-2497 AE Den Haag (NL); EDENS, Luppo, NL-3062 JL Rotterdam (NL)
(74) Representative: Cazemier, Anne Engeline
(86) International application number: PCT/EP2009/056524
(87) International publication number: WO 2009/144269

(56) References cited:
- WO-A-02/46381
- WO-A-02/068623
- WO-A-2005/027953
- DATABASE UniProt [Online] 23 January 2007 (2007-01-23), "SubName: Full=Serine peptidase, family S28, putative;" XP002519166 retrieved from EBI accession no. UNIPROT:A1D9P8 Database accession no. A1D9P8 -& FEDOROVA NATALIE D ET AL: "Genomic islands in the pathogenic filamentous fungus Aspergillus fumigatus." PLOS GENETICS APR 2008, vol. 4, no. 4, April 2008 (2008-04), page e1000046, XP002519164 ISSN: 1553-7404
- DATABASE Geneseq [Online] 28 March 2003 (2003-03-28), "Aspergillus oryzae polynucleotide SEQ ID NO 886." XP002519167 retrieved from EBI accession no. GSN:ABZ51773 Database accession no. ABZ51773
- MITEA C ET AL: "Efficient degradation of gluten by a prolyl endoprotease in a gastrointestinal model: implications for coeliac disease." GUT JAN 2008, vol. 57, no. 1, January 2008 (2008-01), pages 25-32, XP009113711 ISSN: 1468-3288
- VAN DEN BERG MARCO A ET AL: "Genome sequencing and analysis of the filamentous fungus Penicillium chrysogenum." NATURE BIOTECHNOLOGY OCT 2008, vol. 26, no. 10, October 2008 (2008-10), pages 1161-1168, XP002519165 ISSN: 1546-1696 -& DATABASE UniProt [Online] 16 December 2008 (2008-12-16), "SubName: Full=Pc14g00330 protein; Flags: Precursor;" XP002519168 retrieved from EBI accession no. UNIPROT:B6H5K2 Database accession no. B6H5K2 -& DATABASE EMBL [Online] 1 November 2008 (2008-11-01), "Penicillium chrysogenum Wisconsin 54-1255 complete genome, contig Pc00c14" XP002519169 retrieved from EBI accession no. EMBL:AM920429 Database accession no. AM920429

## Description

### Field of the invention

The invention relates to a proline-specific protease. The invention also relates to methods in which the proline-specific protease is used and to uses of the proline-specific protease.

### Background of the invention

Proline-specific proteases are enzymes capable of cleaving a protein or a peptide near or at positions where the protein or peptide contains a prolyl-residue in its chain.

Proline-specific proteases have been proposed for use in a number of applications, for example to minimize the possibility of off-flavours in the preparation of protein hydrolysates, to reduce the antigenicity of such hydrolysates and in the prevention or reduction of haze in beverages.

It is desirable that enzymes that are used in food or beverage applications have a suitable acid pH optimum and, preferably, are not active in the final preparation that they are used to generate. Accordingly, enzymes for use in food or beverage applications may desirably easily be inactivated.

### Summary of the invention

The invention relates to a polypeptide having proline-specific protease activity which comprises the amino acid sequence set out in SEQ ID NO: 3 or an amino acid sequence encoded by the nucleotide sequence of SEQ ID NO: 1 and/or SEQ ID NO: 2, or a variant thereof or a fragment of either thereof.

The proline-specific enzyme of the invention is one which has a pH optimum of from about pH 4 to about pH 5 and which may readily be inactivated. Accordingly, it may be particularly useful in food or beverage applications.

A polypeptide of the invention may have at least about 60% sequence identity with the sequence set out in SEQ ID NO: 3. A polypeptide of the invention may be at least about 150 amino acids in length. A polypeptide having proline-specific protease activity may comprise a functional domain of a polypeptide according to any one of the preceding claims.

The invention also relates to a polynucleotide which comprises:
(a) the nucleotide sequence as set out in SEQ ID NO: 2 or the coding sequence of SEQ ID NO: 1;
(b) a nucleotide sequence which hybridizes selectively with a polynucleotide being the reverse complement of SEQ ID NO: 1 or SEQ ID NO: 2;
(c) a nucleotide sequence having at least about 50% sequence identity with the nucleotide sequence of SEQ ID NO: 1 or SEQ ID NO: 2;
(d) a fragment of a nucleotide sequence as defined in (a), (b) or (c) having at least 100 nucleotides;
(e) a sequence which is degenerate as a result of the genetic code to a sequence as defined in any one of (a), (b), (c) or (d); or
(f) a nucleotide sequence which is the reverse complement of a nucleotide sequence as defined in (a), (b), (c), (d) or (e).

The invention further provides:
- a vector incorporating a polynucleotide sequence of the invention;
- a cell comprising a polypeptide, a polynucleotide or a vector of the invention;
- a method for the preparation of a polypeptide having proline-specific protease activity, which method comprises cultivating a cell of the invention under conditions which allow for expression of said polypeptide and, optionally, recovering the expressed polypeptide;
- a polypeptide obtainable by such a method;
- a composition comprising: (i) a polypeptide of the invention; and, optionally, (ii) an protease which is different from the polypeptide in (i);
- a method for the preparation of a protein hydrolysate, which method comprises contacting a protein substrate with a polypeptide of a composition of the invention;
- use of a polypeptide or a composition of the invention in the preparation of a protein hydrolysate;
- a protein hydrolysate obtainable by the method set out above;
- a food or feed product or a beverage which comprises such a protein hydrolysate;
- a method for the preparation of a food or feed product or a beverage which method comprises incorporating a polypeptide or a composition of the invention during preparation of the food product, feed product or beverage;
- a food or feed product or beverage obtainable by such a method;
- use of a polypeptide or a composition of the invention in the preparation of a food or feed product or a beverage;
- a food or feed product or a beverage obtainable by such a method or use; and
- a polypeptide of the invention for use in the treatment or prophylaxis of a psychiatric disorder or a celiac disease linked disorder.

### Brief description of the drawings

Figure 1 sets out the strategy used to clone the proline-specific protease ZFX.
Figure 2 sets out the sequence of a chromosomal DNA fragment from *Penicillium chrysogenum* CBS 455.95 which contains the gene encoding the proline-specific protease ZFX (SEQ ID NO: 1). The coding sequence (SEQ ID NO: 2) is highlighted and the protein sequence (SEQ ID NO: 3) is indicated.
Figure 3 shows the temperature optimum of the isolated proline-specific protease ZFX.

### Brief description of the sequence listing

SEQ ID NO: 1 sets out the nucleotide sequence of a chromosomal DNA fragment from *Penicillium chrysogenum* CBS 455.95 which contains the gene encoding the proline-specific protease ZFX.
SEQ ID NO: 2 sets out sets out the nucleotide coding sequence of the proline-specific protease ZFX.
SEQ ID NO: 3 sets out the amino acid sequence of the proline-specific protease ZFX.
SEQ ID NO: 4 sets out the direct PCR primer (ZFX-dir) containing 24 nucleotides ZFX coding sequence starting at the ATG start codon, preceded by a 23 nucleotides sequence including a *Pac*I restriction site.
SEQ ID NO: 5 sets out the reverse primer (ZFX-rev) containing nucleotides complementary to the reverse strand of the region downstream of the ZFX coding sequence proceeded by an *Asc*I restriction site.

### Detailed description of the invention

The present invention relates to a new proline-specific protease which has been identified in the fungus *Penicillium chrysogenum.* This is surprising since it was shown in WO02/45524 that a cDNA fragment encoding a proline-specific protease from *A. niger* did not hybridize with genomic DNA isolated from *Penicillium chrysogenum* (see Example 11 and Table 6 in WO 02/45524).

The new enzyme described herein has an acid pH optimum and may be substantially, or preferably, entirely inactivated via, for example, a heating step (such as a standard pasteurization process). Thus the enzyme is suited for use in food applications which typically are acid environments and where it is highly desirable that the final (food) product has substantially no residual enzyme activity.

Also, the present invention meets the demand for a proline-specific protease that can be produced in high amounts. Preferably, such a proline-specific protease is secreted from the host cell. Active secretion is of paramount importance for an economically viable production process since it enables the recovery of the enzyme in an almost pure form without going through cumbersome purification processes. Overexpression of such an actively secreted proline-specific protease by a food grade fungal host such as *Aspergillus*, yields a food grade enzyme and a cost effective production process, and is therefore preferable. Processes are disclosed for the production of proline-specific protease in large amounts by the food-grade production host *Aspergillus niger.*

From an economic point of view there exists a clear need for an improved means of producing proline-specific proteases in high quantities and in a relatively pure form. We show here that a preferred way of doing this is via the overproduction of such a proline-specific protease using recombinant DNA techniques. A particulary preferred way of doing this is via the overproduction of a fungal derived proline-specific protease and a most preferred way of doing this is via the overproduction of a *Penicillium* derived proline-specific protease. To enable the latter production route unique sequence information of a *Penicillium* derived proline-specific protease is essential. More preferable the whole nucleotide sequence of the encoding gene has to be available.

Once the new enzyme has been made available in large quantities and in a relatively pure form, food stuffs (like bread, pasta or noodles) or protein hydrolysates with improved textural, organoleptic and/or immunological properties can be prepared in a food grade and economic way. The enzyme can also potentially be used as a preservative. Additionally, the enzyme may be used in the preparation of beverages, in particular in a method for the prevention or reduction of haze in a beverage.

Herein, proline-specific protease activity is defined as protease activity that cleaves peptide bonds involving a proline residue in a protein and/or a peptide. A protein may generally be considered for the purposes of this invention to comprise at more than about 30 amino acids. A peptide may generally be considered for the purposes of this invention to comprise about 30 or less amino acids.

Accordingly, a proline-specific protease herein may have proline-specific endoprotease activity and/or proline-specific oligopeptidase (EC 3.4.21.26). Preferably, a proline-specific protease of the invention, such as a proline-specific endoprotease and/or a proline-specific oligopeptidase, is one which hydrolyses a protein and/or a peptide at a location where the protein or peptide contains a proline-residue.

In the invention, a proline-specific protease is preferably one which hydrolyses the peptide bond at the carboxylterminal end of proline residues. However, a prolyl-specific protease that cuts prolyl-residues at their NH₂-terminus is for example described in a publication in Nature of 15 January 1998, Vol.391, p.301-304.

In this text, the terms prolyl-specific endoprotease, proline-specific endoprotease, proline-specific endopeptidase, proline-specific oligopeptidase, prolyl-specific oligopeptidase, prolyl oligopeptidease, protein/peptide having a prolyl-specific activity or similar expressions are used interchangeably.

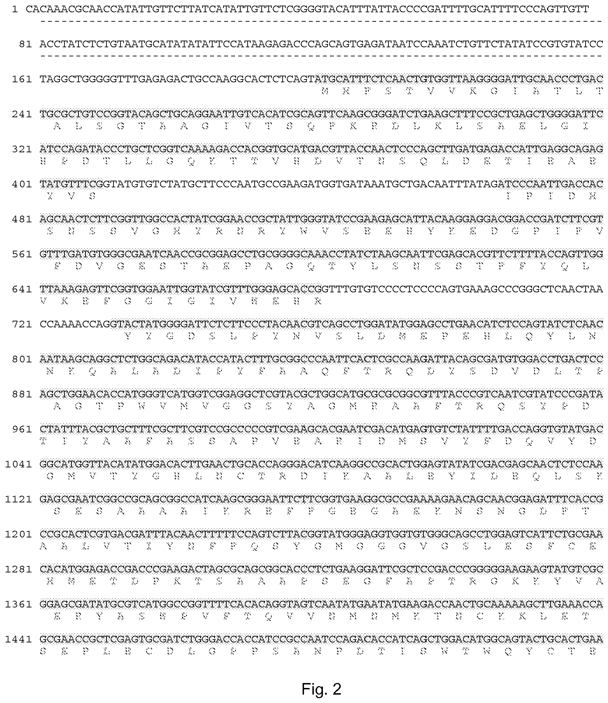

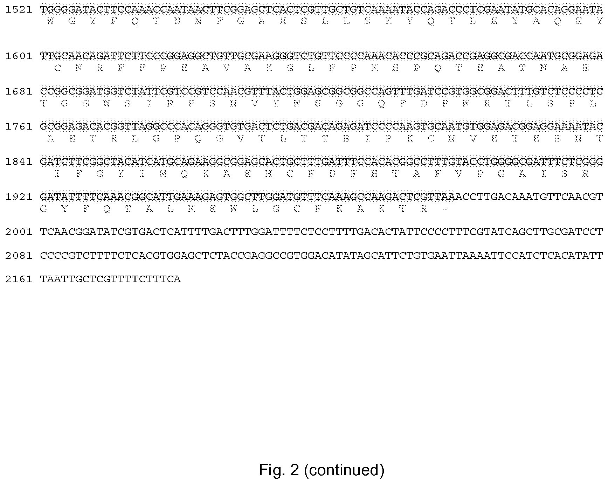

The present invention provides polynucleotides encoding a proline-specific protease, herein referred to as "ZFX", having an amino acid sequence according to SEQ ID NO: 3 or a variant sequence thereof (for example a sequence which is functionally equivalent to that of SEQ ID NO: 3) or a sequence which is a fragment of either thereof. The amino acid sequence is also set out in Figure 2.

The sequence of the gene encoding the ZFX proline-specific protease was determined by sequencing the genome of *Penicillium chrysogenum.* The invention provides polynucleotide sequences comprising the gene encoding the ZFX proline-specific protease as well as its coding sequence. Accordingly, the invention relates to an isolated polynucleotide comprising the nucleotide sequence according to SEQ ID NO: 1 or 2 and to variants, such as functional equivalents, thereof. The nucleotide sequences are also set out in Figure 2.

In particular, the invention relates to an isolated polynucleotide which is capable of hybridizing selectively, for example under stringent conditions, preferably under highly stringent conditions, with the reverse complement of a polynucleotide comprising the coding sequence of SEQ ID NO: 1 or the sequence set out in SEQ ID NO: 2.

Advantageously, such polynucleotides according to the invention (and the polypeptides encoded thereby) may be obtainable and/or obtained from a fungus, in particular from a fungus of the division Ascomycota, such as from the subdivision Pezizomycotina, for example from the class Eurotiomycetes, such as from the order Eurotiales, in particular from the family Trichocomaceae, such as from the genus *Penicillium,* preferably from the species *Penicillium chrysogenum.*

More specifically, the invention relates to a polynucleotide comprising or consisting essentially of a nucleotide sequence according to coding sequence of SEQ ID NO: 1 or the sequence set out in SEQ ID NO: 2.

The invention also relates to an isolated polynucleotide comprising or consisting essentially of a sequence which encodes at least one functional domain of a polypeptide according to SEQ ID NO: 3 or a variant, such as a functional equivalent, thereof or a fragment of either thereof.

As used herein, the terms "gene" and "recombinant gene" refer to nucleic acid molecules which may be isolated from chromosomal DNA, which include an open reading frame encoding a protein, e.g. a *Penicillium chrysogenum* proline-specific protease according to the present invention.

A gene may include coding sequences, non-coding sequences, introns and/or regulatory sequences. Moreover, the term "gene" may refer to an isolated nucleic acid molecule as defined herein.

A nucleic acid molecule of the present invention, such as a nucleic acid molecule having the nucleotide sequence of coding sequence of SEQ ID NO: 1 or the sequence set out in SEQ ID NO: 2 or a functional equivalent of either thereof, can be isolated using standard molecular biology techniques and the sequence information provided herein. For example, using all or portion of the nucleic acid sequence of SEQ ID NO: 1 or 2 as a hybridization probe, nucleic acid molecules according to the invention can be isolated using standard hybridization and cloning techniques (e. g., as described in Sambrook, J., Fritsh, E. F., and Maniatis, T. Molecular Cloning: A Laboratory Manual.2nd, ed., Cold Spring Harbor Laboratory, Cold Spring Harbor Laboratory Press, Cold Spring Harbor, NY, 1989).

Moreover, a nucleic acid molecule encompassing all or a portion of SEQ ID NO: 1 or 2 can be isolated by the polymerase chain reaction (PCR) using synthetic oligonucleotide primers designed based upon the sequence information contained in SEQ ID NO: 1 or 2.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Herein is described the isolation and cloning of the fragment of SEQ ID NO: 1. The fragment was amplified by PCR using the oligonucleotide primers set out in SEQ ID NOs: 4 and 5.

Furthermore, oligonucleotides corresponding to or hybridizable to a nucleotide sequence according to the invention can be prepared by standard synthetic techniques, e.g., using an automated DNA synthesizer.

In a preferred embodiment, an isolated nucleic acid molecule of the invention comprises the nucleotide sequence which is the coding sequence of SEQ ID NO: 1 or the sequence set out in SEQ ID NO: 2. The sequence of SEQ ID NO: 2 corresponds to the coding region of the *Penicillium chrysogenum* proline-specific protease cDNA. This cDNA comprises sequences encoding the *Penicillim chrysogenum* proline-specific protease polypeptide according to SEQ ID NO: 3.

In another preferred embodiment, an isolated nucleic acid molecule of the invention comprises a nucleic acid molecule which is the reverse complement of the nucleotide sequence shown in SEQ ID NO: 1 or 2 or a variant, such as a functional equivalent, of such a nucleotide sequence.

A nucleic acid molecule which is complementary to another nucleotide sequence is one which is sufficiently complementary to the other nucleotide sequence such that it can hybridize to the other nucleotide sequence thereby forming a stable duplex.

One aspect of the invention pertains to isolated nucleic acid molecules that encode a polypeptide of the invention or a variant, such as a functional equivalent thereof, for example a biologically active fragment or domain, as well as nucleic acid molecules sufficient for use as hybridization probes to identify nucleic acid molecules encoding a polypeptide of the invention and fragments of such nucleic acid molecules suitable for use as PCR primers for the amplification or mutation of nucleic acid molecules.

One example of a biologically active fragment or domain is a fragment or domain which is capable of at least cleaving a detectable group from a (synthetic) substrate which (synthetic) substrate comprises a "proline coupled to a means for detection" at the carboxy terminus of the (synthetic) substrate and wherein said means for detection is coupled to the carboxy side of the proline via a chemical bond that is cleavable by a protease. The experimental part provides examples of suitable (synthetic) substrates. An example of a means for detection is a chromogenic or fluorogenic group.

A polynucleotide according to the invention may be "isolated". In the context of this invention, an "isolated polynucleotide" or "isolated nucleic acid" is a DNA or RNA that is not immediately contiguous with both of the coding sequences with which it is immediately contiguous (one on the 5' end and one on the 3' end) in the naturally occurring genome of the organism from which it is derived. Thus, in one embodiment, an isolated nucleic acid includes some or all of the 5' non-coding (e.g., promotor) sequences that are immediately contiguous to the coding sequence. The term therefore includes, for example, a recombinant DNA that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA that is part of a hybrid gene encoding an additional polypeptide that is substantially free of cellular material, viral material, or culture medium (when produced by recombinant DNA techniques), or chemical precursors or other chemicals (when chemically synthesized). Moreover, an "isolated nucleic acid fragment" is a nucleic acid fragment that is not naturally occurring as a fragment and would not be found in the natural state.

As used herein, the terms "polynucleotide" or "nucleic acid molecule" are intended to include DNA molecules (e.g., cDNA or genomic DNA) and RNA molecules (e.g., mRNA) and analogs of the DNA or RNA generated using nucleotide analogs. That is to say, a polynucleotide of the invention may comprise DNA and/or RNA. A polynucleotide according to the invention may thus be a DNA sequence or an RNA sequence. The nucleic acid molecule can be single-stranded or double-stranded, but preferably is double-stranded DNA. The nucleic acid may be synthesized using synthetic or modified nucleotides including peptide nucleic acids, oligonucleotide analogs or derivatives (e.g., inosine, methylphosphonate or phosphorothioate nucleotides and addition of acridine or polylysine chains at the 3' and/or 5' ends of the molecule). Such nucleotides and oligonucleotides can be used, for example, to prepare nucleic acids that have altered base-pairing abilities or increased resistance to nucleases. For the purposes of the present invention, it is to be understood that the poly-nucleotides described herein may be modified by any method available in the art.

Another embodiment of the invention provides an isolated nucleic acid molecule which is antisense to the ZFX nucleic acid molecule, e.g., the coding strand of a ZFX nucleic acid molecule. Also included within the scope of the invention are the complement strands of the nucleic acid molecules described herein.

The sequence information as provided herein should not be so narrowly construed as to require inclusion of erroneously identified bases. The specific sequences disclosed herein can be readily used to isolate the complete gene from a filamentous fungus, in particular from *Penicillium chrysogenum* which in turn can easily be subjected to further sequence analyses thereby identifying sequencing errors.

Unless otherwise indicated, all nucleotide sequences determined by sequencing a DNA molecule herein were determined using an automated DNA sequencer and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule.

The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

The person skilled in the art is capable of identifying such erroneously identified bases and knows how to correct for such errors.

A nucleic acid molecule according to the invention may comprise only a portion or a fragment of the nucleic acid sequence shown in SEQ ID NO: 1, for example a fragment which can be used as a probe or primer or a fragment encoding a portion of a ZFX protein.

The nucleotide sequence determined from the cloning of the ZFX gene and cDNA allows for the generation of probes and primers designed for use in identifying and/or cloning other ZFX family members, as well as ZFX homologues from other species.

The probe/primer typically comprises a substantially purified oligonucleotide which typically comprises a region of nucleotide sequence that hybridizes, for example under highly stringent conditions, to at least about 12 to 15, preferably about 18 to 20, preferably about 22 to 25, more preferably about 30, 35, 40, 45, 50, 55, 60, 65, or 75 or more consecutive nucleotides of a nucleotide sequence shown in SEQ ID NO:1 or 2 (or of a functional equivalent thereof or of the reverse complement of any thereof.

Probes based on the ZFX nucleotide sequences can be used to detect transcripts or genomic ZFX sequences encoding the same or homologous proteins for instance in other organisms. In preferred embodiments, the probe further comprises a label group attached thereto, e.g., the label group can be a radioisotope, a fluorescent compound, an enzyme, or an enzyme cofactor. Such probes can also be used as part of a diagnostic test kit for identifying cells which express a ZFX protein.

The terms "homology", "sequence identity" and the like are used interchangeably herein. For the purpose of this invention, it is defined here that in order to determine the degree of sequence identity shared by two amino acid sequences or by two nucleic acid sequences, the sequences are aligned for optimal comparison purposes (e.g., gaps can be introduced in the sequence of a first amino acid or nucleic acid sequence for optimal alignment with a second amino or nucleic acid sequence). Such alignment may be carried out over the full lengths of the sequences being compared. Alternatively, the alignment may be carried out over a shorter length, for example over about 20, about 50, about 100 or more nucleic acids/based or amino acids.

The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The degree of identity shared between sequences is typically expressed in term of percentage identity between the two sequences and is a function of the number of identical positions shared by the sequences (i.e., % identity = number of identical positions/total number of positions (i.e. overlapping positions) x 100). The two sequences may be compared over their entire lengths.

The skilled person will be aware of the fact that several different computer programs are available to determine the homology between two sequences. For instance, a comparison of sequences and determination of percent identity between two sequences can be accomplished using a mathematical algorithm.

The percent identity two amino acid or nucleotide sequence may be determined using, for example, the algorithm of E. Meyers and W. Miller (CABIOS, 4:11-17 (1989) which has been incorporated into the ALIGN program (version 2.0) (available at the ALIGN Query using sequence data of the Genestream server IGH Montpellier France http://vega.igh.cnrs.fr/bin/align-guess.cgi) using a PAM120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the BLASTN, BLASTP and BLASTX programs (version 2.0) of Altschul, et al. (1990) J. Mol. Biol. 215:403-10. BLAST nucleotide searches in the nucleotide databases can be performed with the BLASTN program to obtain nucleotide sequences homologous to ZFX nucleic acid molecules of the invention. BLAST searches with a translated nucleotide sequence in the protein databases can be performed with the BLASTX program to obtain amino acid sequences homologous to the translated ZFX gene of the invention. Alternatively, for protein sequence comparison with the protein databases the BLASP program can be used with matrix Blosum 62, an expected threshold = 10, word length = 3, gap existence costs of 11 and gap extension costs of 1. When utilizing BLAST programs, the default parameters of the respective programs (e.g., BLASTX, BLASTP and BLASTN) can be used. See the homepage of the National Center for Biotechnology Information at http://www.ncbi.nlm.nih.gov/ for all relevant information on homology searches in public databases.

The BLASTP and BLAST N algorithms can be used to calculate sequence identity or to line up sequences (such as identifying equivalent or corresponding sequences, for example on their default settings).

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighborhood word score threshold. These initial neighborhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program from DNA-DNA comparison uses as defaults a word length (W) of 11, expectation (E) of 10, and a comparison of both strands. The BLASTP program for protein-protein comparison uses as defaults a word length (W) of 3, the BLOSUM62 scoring matrix, a gap existence penalty of 11 with a gap extension penalty of 1, and an expectation (E) of 10.

The BLAST algorithm performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Also, a peptide motif can be used to identify genes that code for proteins containing this peptide motif. Instead of one peptide motif, also a combination of two or more peptide motifs can be used to identify genes coding for proteins containing the peptide motifs. When one or several peptide motifs coding for specific proline-specific proteases are identified it is thus possible to identify genes coding for proline-specific proteases using one or a combination of several of these peptide motifs. Proline-specific proteases are used as an example how such genes may be identified, but the methods described are generally applicable. A peptide motif can be used for a search in translated DNA sequences from a DNA databank or protein sequences from a protein sequence databank using a program like Patscan (http://www-unix.mcs.anl.gov/compbio/PatScan/HTML/). The amino acid sequence has to be entered in a special format that is described on the website. Another method that can be performed is to use the sequence of the motif for a search in translated DNA sequences from a DNA databank or protein sequences from a protein sequence databank using a program like http://myhits.isb-sib.ch/cgi-bin/. For this program the motif is entered in the search field in the so called Prosite format, and databases are searched for the presence of the motif in the protein sequence or in the translated DNA sequence. This method can be used to identify fungal genes that encode useful proline-specific proteases. The genes that are identified using one of these methods can than be translated into a protein sequence using programs known to those skilled in the art, and be inspected for the presence of a signal sequence at their amino-terminus. For detecting a signal sequence one can use a program like SignalP (http://www.cbs.dtu.dk/services/SignalP/). Looking for a protein sequence that contains both the consensus sequences and a predicted signal sequence gives a large advantage for the industrial production of such an enzyme.

Another possibility to identify proline-specific protease genes using peptide motifs is to design oligonucleotide primers based on the back-translation of the amino acid sequence of the motif into a nucleotide sequence with preferred codon usage from the organism in which one wants to identify a proline-specific protease gene, and using this oligonucleotide for hybridization to a gene library, or in a PCR primer on a reverse transcribed mRNA pool. Using a peptide sequence motif, it is also possible to isolate genes encoding proline-specific protease when the gene sequence is unknown. Methods have been described in literature to design degenerate oligonucleotide primers that can be used for this purpose (Sambrook et al. (1989) Molecular cloning: a laboratory manual. Cold Spring Harbor Laboratory Press). Also, methods to isolate genes from an organism, using a degenerate oligonucleotide as probe or primer, have been described.

Oligonucleotides that code for the peptide motif are useful for isolation of the genes encoding proline-specific protease properties. The degeneracy of such a group of oligonucleotides may be decreased by the introduction of inosine (I) bases at positions where the nucleotide is not known. Additionally, positions where both cytosine (C) and thymidine (T) bases are possible may be replaced by uracil (U), and at positions where both adenine (A) and guanine (G) are possible only guanine may be introduced, in order to decrease degeneracy with only a small effect on specificity of the oligonucleotide primer. Furthermore, for screening the presence of genes encoding proline-specific proteases in organisms of which the codon preference is known, the degeneracy of the oligonucleotide can be further decreased by taking the codon preference into account in the design of the oligonucleotide. A person skilled in the art will know how to do this. Furthermore, all possible combinations of oligonucleotide primers, without degeneracy, may be synthesized separately and used in individual screening experiments.

First, a genomic, cDNA or EST library is constructed from the species of interest in a universal vector. Suitable methods for library construction are described in literature (Sambrook et al. (1989) Molecular cloning: a laboratory manual. Cold Spring Harbor Laboratory Press). Second, a degenerate oligonucleotide described above is used in a PCR reaction together with one universal oligonucleotide that primes in the vector, at the border of the recombinant DNA insert, on DNA isolated from the library. Useful strategies have been described in literature for the isolation of a desired gene when only a single degenerate oligonucleotide primer is available (e.g. Minambres et al. (2000) Biochem. Biophys. Res. Commun. 272, 477-479; PCR technology (1989) Ed. H.A. Erlich pp. 99-104, Stockton Press). Third, the PCR amplified fragment is then labeled and used as probe for the screening of the library by conventional means. The full length gene can than be sub-cloned into an expression vector suitable for over-expression of the proline-specific protease in a desired production host organism.

In a different approach, when no library is available from the species that is screened for the presence of a gene encoding a proline-specific protease, part of the gene can be amplified by PCR with different degenerate primers, or with 3'-RACE using a single degenerate primer. For this, RNA is isolated from the species of interest and used in a 3'-RACE reaction using a single degenerate primer as gene specific primer. The amplification of part of an unknown cDNA using one degenerate oligonucleotide and one universal primer, by 3'-RACE, has been described previously (WO99/38956).

The traditional method to isolate a full-length gene using the information from only a small peptide is hybridization of a labeled degenerate oligonucleotide to filters on which a library is replicated. Methods describing the screening of gene libraries using degenerate oligonucleotides, and methods to calculate or determine the optimal hybridization conditions of these oligonucleotides, have been extensively described in literature (Sambrook et al.(1989)). The oligonucleotides described above may be used for this method to isolate genes encoding a proline-specific protease from different species.

In a variation to this method, a partial gene library can be constructed first. For this, DNA is fractionated, after which fragments of DNA containing the gene coding for a proline-specific protease are detected by hybridization to the labeled oligonucleotides described above. These fragments are isolated and used in the construction of a partial gene library enriched in the gene coding for a proline-specific protease. This library can than be screened by conventional means. For this method, genomic DNA is first digested with restriction enzymes before fractionation by gel-electrophoresis, while cDNA can be fractionated directly.

A different method to isolate the gene coding for a proline-specific protease is by using antibodies raised against any of the peptides of the consensus sequence. Antibodies may be monoclonal or polyclonal. Methods describing the production of antibodies specific for small peptides have been extensively described in literature (Harlow, E and Lane, D (1988) Antibodies; a laboratory manual, ISBN 0-87969-314. -2).

Expression libraries can be constructed from the species of interest, by cloning cDNA or genomic DNA into a vector suitable for expressing the insert in a convenient host, such as *E. coli* or a yeast. Expression vectors may or may not be based on phage lambda. Immuno-detection of antigens produced by expression libraries, and methods describing the purification of specific clones expressing the antigen has been published. Using an antibody specific for any of the peptides of the consensus motif, it is possible to isolate the gene encoding a proline-specific protease encompassing this motif, using this method.

In effect, many different methods may be used to isolate a gene coding for a proline-specific protease when the information described in this invention is taken into account. The advantage of using the peptide motif sequence information over prior art methods is the speed and relative ease with which a new gene coding for an active proline-specific protease can be identified. The use of the sequence information gives an indication of the value of a new proline-specific protease in applications in food industry, without performing laborious testing of all possible in direct application experiments.

As used herein, the term "selectively hybridizing" and similar terms are intended to describe conditions for hybridization and washing under which nucleotide sequences at least about 60%, at least about 70%, at least about 80%, more preferably at least about 85%, even more preferably at least about 90%, preferably at least 95%, more preferably at least about 98% or more preferably at least about 99% homologous to each other typically remain hybridized to each other. That is to say, such hybridizing sequences may share at least about 50%, at least about 60%, at least about 70%, at least about 80%, more preferably at least about 85%, even more preferably at least about 90%, more preferably at least 95%, more preferably at least 98% or more preferably at least about 99% sequence identity.

Thus, a nucleotide sequence which is capable of selectively hybridizing to the reverse complement of the sequence of SEQ ID NO: 1 and/or SEQ ID NO: 2 is included in the invention and will generally have at least 50% or 60%, at least 70%, at least 80%, at least 90%, at least 95%, at least 98% or at least 99% sequence identity to the coding sequence of SEQ ID NO:1 and/or SEQ ID NO: 2 over a region of at least 60, preferably at least 100, more preferably at least 200 contiguous nucleotides or most preferably over the full length of SEQ ID NO: 1 and/or SEQ ID NO: 2.

Likewise, a nucleotide which encodes an active proline-specific protease and which is capable of selectively hybridizing to a fragment of a complement of the DNA coding sequence of SEQ ID NO: 1 and/or SEQ ID NO: 2, is also embraced by the invention. Any combination of the above mentioned degrees of identity and minimum sizes may be used to define poly-nucleotides of the invention, with the more stringent combinations (i.e. higher identity over longer lengths) being preferred. Thus, for example, a polynucleotide which is at least 80% or 90% identical over 60, preferably over 100 nucleotides, forms one aspect of the invention, as does a polynucleotide which is at least 90% identical over 200 nucleotides.

A preferred, non-limiting example of such hybridization conditions is hybridization in 6X sodium chloride/sodium citrate (SSC) at about 45°C, followed by one or more washes in 1 X SSC, 0.1 % SDS at 50°C, preferably at 55°C, preferably at 60°C and even more preferably at 65°C.

Highly stringent conditions include, for example, hybridization at 68°C in 5x SSC/5x Denhardt's solution / 1.0% SDS and washing in 0.2x SSC/0.1% SDS at room temperature. Alternatively, washing may be performed at 42°C.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.).

Of course, a polynucleotide which hybridizes only to a poly A sequence (such as the 3' terminal poly(A) tract of mRNAs), or to a complementary stretch of T (or U) resides, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any double-stranded cDNA clone).

In a typical approach, cDNA libraries constructed from other organisms, e.g. a filamentous fungi, in particular from the micro-organism family Trichomaceae, for example from the genus *Aspergillus* or *Penicillium* can be screened.

For example, *Penicillium* strains can be screened for homologous ZFX polynucleotides by Northern blot analysis. Upon detection of transcripts homologous to polynucleotides according to the invention, cDNA libraries can be constructed from RNA isolated from the appropriate strain, utilizing standard techniques well known to those of skill in the art. Alternatively, a total genomic DNA library can be screened using a probe capable of hybridizing to a ZFX polynucleotide according to the invention.

Homologous gene sequences can be isolated, for example, by performing PCR using two degenerate oligonucleotide primer pools designed on the basis of nucleotide sequences as taught herein.

The template for the reaction can be cDNA obtained by reverse transcription of mRNA prepared from strains known or suspected to express a polynucleotide according to the invention. The PCR product can be subcloned and sequenced to ensure that the amplified sequences represent the sequences of a new ZFX nucleic acid sequence, or a functional equivalent thereof.

The PCR fragment can then be used to isolate a full-length cDNA clone by a variety of known methods. For example, the amplified fragment can be labelled and used to screen a bacteriophage or cosmid cDNA library. Alternatively, the labelled fragment can be used to screen a genomic library.

PCR technology also can be used to isolate full-length cDNA sequences from other organisms. For example, RNA can be isolated, following standard procedures, from an appropriate cellular or tissue source. A reverse transcription reaction can be performed on the RNA using an oligonucleotide primer specific for the most 5' end of the amplified fragment for the priming of first strand synthesis.

The resulting RNA/DNA hybrid can then be "tailed" (e.g., with guanines) using a standard terminal transferase reaction, the hybrid can be digested with RNase H, and second strand synthesis can then be primed (e.g., with a poly-C primer). Thus, cDNA sequences upstream of the amplified fragment can easily be isolated. For a review of useful cloning strategies, see e.g.,Sambrook *et al*., supra; and Ausubel *et al*., supra.

Another aspect of the invention pertains to vectors, including cloning and expression vectors, comprising a polynucleotide of the invention encoding a ZFX protein or a functional equivalent thereof and methods of growing, transforming or transfecting such vectors in a suitable host cell, for example under conditions in which expression of a polypeptide of the invention occurs. As used herein, the term "vector" refers to a nucleic acid molecule capable of transporting another nucleic acid to which it has been linked.

Polynucleotides of the invention can be incorporated into a recombinant replicable vector, for example a cloning or expression vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below.

The vector into which the expression cassette or polynucleotide of the invention is inserted may be any vector which may conveniently be subjected to recombinant DNA procedures, and the choice of the vector will often depend on the host cell into which it is to be introduced.

A vector according to the invention may be an autonomously replicating vector, i. e. a vector which exists as an extra-chromosomal entity, the replication of which is independent of chromosomal replication, e. g. a plasmid. Alternatively, the vector may be one which, when introduced into a host cell, is integrated into the host cell genome and replicated together with the chromosome (s) into which it has been integrated.

One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid" and "vector" can be used interchangeably herein as the plasmid is the most commonly used form of vector. However, the invention is intended to include such other forms of expression vectors, such as cosmid, viral vectors (e.g., replication defective retroviruses, adenoviruses and adeno-associated viruses) and phage vectors which serve equivalent functions.

Vectors according to the invention may be used *in vitro,* for example for the production of RNA or used to transfect or transform a host cell.

A vector of the invention may comprise two or more, for example three, four or five, polynucleotides of the invention, for example for overexpression.

The recombinant expression vectors of the invention comprise a nucleic acid of the invention in a form suitable for expression of the nucleic acid in a host cell, which means that the recombinant expression vector includes one or more regulatory sequences, selected on the basis of the host cells to be used for expression, which is operably linked to the nucleic acid sequence to be expressed.

Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell), i.e. the term "operably linked" refers to a juxtaposition wherein the components described are in a relationship permitting them to function in their intended manner. A regulatory sequence such as a promoter, enhancer or other expression regulation signal "operably linked" to a coding sequence is positioned in such a way that expression of the coding sequence is achieved under condition compatible with the control sequences or the sequences are arranged so that they function in concert for their intended purpose, for example transcription initiates at a promoter and proceeds through the DNA sequence encoding the polypeptide.

The invention thus provides a vector, which may be an expression vector, for example wherein the polynucleotide sequence is operably linked with a regulatory sequence, allowing for expression of the polynucleotide sequence in a cell, for example the cell of a filamentous fungus.

The term "regulatory sequence" is intended to include promoters, enhancers and other expression control elements (e.g., polyadenylation signal). Such regulatory sequences are described, for example, in Goeddel; Gene Expression Technology: Methods in Enzymology 185, Academic Press, San Diego, CA (1990).

The term regulatory sequences includes those sequences which direct constitutive expression of a nucleotide sequence in many types of host cells and those which direct expression of the nucleotide sequence only in a certain host cell (e.g. tissue-specific regulatory sequences).

A vector or expression construct for a given host cell may thus comprise the following elements operably linked to each other in a consecutive order from the 5'-end to 3'-end relative to the coding strand of the sequence encoding the polypeptide of the first invention: (1) a promoter sequence capable of directing transcription of the nucleotide sequence encoding the polypeptide in the given host cell ; (2) optionally, a signal sequence capable of directing secretion of the polypeptide from the given host cell into a culture medium; (3) a DNA sequence of the invention encoding a mature and preferably active form of a polypeptide having proline-specific protease activity; and preferably also (4) a transcription termination region (terminator) capable of terminating transcription downstream of the nucleotide sequence encoding the polypeptide.

Downstream of the nucleotide sequence according to the invention there may be a 3' untranslated region containing one or more transcription termination sites (e. g. a terminator). The origin of the terminator is less critical. The terminator can, for example, be native to the DNA sequence encoding the polypeptide. However, preferably a yeast terminator is used in yeast host cells and a filamentous fungal terminator is used in filamentous fungal host cells. More preferably, the terminator is endogenous to the host cell (in which the nucleotide sequence encoding the polypeptide is to be expressed). In the transcribed region, a ribosome binding site for translation may be present. The coding portion of the mature transcripts expressed by the constructs will include a translation initiating AUG at the beginning and a termination codon appropriately positioned at the end of the polypeptide to be translated.

Enhanced expression of the polynucleotide of the invention may also be achieved by the selection of heterologous regulatory regions, e. g. promoter, secretion leader and/or terminator regions, which may serve to increase expression and, if desired, secretion levels of the protein of interest from the expression host and/or to provide for the inducible control of the expression of a polypeptide of the invention.

The invention thus provides, *inter alia*, a vector, which may be an expression vector, for example wherein the polynucleotide sequence is operably linked with a regulatory sequence, allowing for expression of the polynucleotide sequence in a cell, for example the cell of a filamentous fungus.

It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, encoded by nucleic acids as described herein (e.g. ZFX proteins, mutant forms of ZFX proteins, fragments, variants or functional equivalents thereof, fusion proteins, etc.).

Promoters/enhancers and other expression regulation signals may be selected to be compatible with the host cell for which the expression vector is designed. For example prokaryotic promoters may be used, in particular those suitable for use in E.coli strains. When expression of the polypeptides of the invention is carried out in mammalian cells, mammalian promoters may be used. Tissues-specific promoters, for example hepatocyte cell-specific promoters, may also be used. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, herpes simplex virus promoters or adenovirus promoters.

Suitable yeast promoters include the S. cerevisiae GAL4 and ADH promoters and the S. pombe nmt1 and adh promoter. Mammalian promoters include the metallothionein promoter which can be induced in response to heavy metals such as cadmium. Viral promoters such as the SV40 large T antigen promoter or adenovirus promoters may also be used. All these promoters are readily available in the art.

Mammalian promoters, such as ß-actin promoters, may be used. Tissue-specific promoters, in particular endothelial or neuronal cell specific promoters (for example the DDAHI and DDAHII promoters), are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). Viral promoters are readily available in the art.

The recombinant expression vectors of the invention can be designed for expression of proteins in prokaryotic or eukaryotic cells. For example, ZFX proteins can be expressed in bacterial cells such as *E. coli,* insect cells (using baculovirus expression vectors) yeast cells, fungal cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

For most filamentous fungi and yeast, the vector or expression construct is preferably integrated in the genome of the host cell in order to obtain stable transformants. However, for certain yeasts and fungi also suitable episomal vectors are available into which the expression construct can be incorporated for stable and high level expression, examples thereof include vectors derived from the 2µ, CEN and pKD1 plasmids of *Saccharomyces* and *Kluyveromyces*, respectively, or vectors containing an AMA sequence (e.g. AMA1 from *Aspergillus*). In case the expression constructs are integrated in the host cells genome, the constructs are either integrated at random loci in the genome, or at predetermined target loci using homologous recombination, in which case the target loci preferably comprise a highly expressed gene. A highly expressed gene is a gene whose mRNA can make up at least 0.01% (w/w) of the total cellular mRNA, for example under induced conditions, or alternatively, a gene whose gene product can make up at least 0.2% (w/w) of the total cellular protein, or, in case of a secreted gene product, can be secreted to a level of at least 0.05 g/l.

Accordingly, expression vectors useful in the present invention include chromosomal-, episomal- and virus-derived vectors e.g., vectors derived from bacterial plasmids, bacteriophage, yeast episome, yeast chromosomal elements, viruses such as baculoviruses, papova viruses, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids.

The nucleotide insert should be operatively linked to an appropriate promoter. Aside from the promoter native to the gene encoding the polypeptide of the invention, other promoters may be used to direct expression of the polypeptide of the invention. The promoter may be selected for its efficiency in directing the expression of the polypeptide of the invention in the desired expression host. Examples of promoters which may be useful in the invention include the phage lambda PL promoter, the *E. coli* lac, trp and tac promoters, the SV40 early and late promoters and promoters of retroviral LTRs, to name a few. Other suitable promoters will be known to the skilled person. In a specific embodiment, promoters are preferred that are capable of directing a high expression level of a proline-specific protease in a fungus or yeast. Such promoters are known in the art.

A variety of promoters can be used that are capable of directing transcription in the host cells of the invention. Preferably the promoter sequence is derived from a highly expressed gene. Examples of preferred highly expressed genes from which promoters are preferably derived and/or which are comprised in preferred predetermined target loci for integration of expression constructs, include but are not limited to genes encoding glycolytic enzymes such as triose-phosphate isomerases (TPI),glyceraldehyde-phosphate dehydrogenases (GAPDH), phosphoglycerate kinases (PGK), pyruvate kinases (PYK or PKI), alcohol dehydrogenases (ADH), as well as genes encoding amylases, glucoamylases, proteases, xylanases, cellobiohydrolases,β-galactosidases, alcohol (methanol) oxidases, elongation factors and ribosomal proteins. Specific examples of suitable highly expressed genes include e. g. the *LAC4* gene from *Kluyveromyces* sp., the methanol oxidase genes (*AOX* and MOX) from *Hansenula* and *Pichia*, respectively, the glucoamylase (*glaA*) genes from *A. niger* and *A. awamori*, the *A. oryzae* TAKA-amylase gene, the *A. nidulans gpdA* gene and the *T. reesei* cellobiohydrolase genes.

Examples of strong constitutive and/or inducible promoters which are preferred for use in fungal expression hosts are those which are obtainable from the fungal genes for xylanase (*xlnA*), phytase, ATP-synthetase, subunit 9 (*oliC*), triose phosphate isomerase (*tpi*), alcohol dehydrogenase (*AdhA*), α-amylase (amy), amyloglucosidase (AG-from the *gla*A gene), acetamidase (*amd*S) and glyceraldehyde-3-phosphate dehydrogenase (*gpd*) promoters.

Examples of strong yeast promoters are those obtainable from the genes for alcohol dehydrogenase, lactase, 3-phosphoglycerate kinase and triosephosphate isomerase.

Examples of strong bacterial promoters are the α-amylase and *SPo*2 promoters as well as promoters from extracellular protease genes.

Promoters suitable for plant cells include nopaline synthase (nos), octopine synthase (*ocs*), mannopine synthase (*mas*), ribulose small subunit (rubisco ssu), histone, rice actin, phaseolin, cauliflower mosaic virus (CMV) 35S and 19S and circovirus promoters.

All of the above-mentioned promoters are readily available in the art.

The vector may further include sequences flanking the polynucleotide giving rise to RNA which comprise sequences homologous to eukaryotic genomic sequences or viral genomic sequences. This will allow the introduction of the polynucleotides of the invention into the genome of a host cell. In particular, a plasmid vector comprising the expression cassette flanked by fungal sequences can be used to prepare a vector suitable for delivering the polynucleotides of the invention to a fungal cell. Transformation techniques using these fungal vectors are known to those skilled in the art.

The vector may contain a polynucleotide of the invention oriented in an antisense direction to provide for the production of antisense RNA. The vector may contain a polynucleotide of the invention oriented in an antisense direction to provide for the production of antisense RNA. This may be used to reduce, if desirable, the levels of expression of the polypeptide.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-percipitation, DEAE-dextran-mediated transfection, transduction, infection, lipofection, cationic lipidmediated transfection or electroporation. Suitable methods for transforming or transfecting host cells can be found in Sambrook, et al. *(Molecular Cloning: A Laboratory Manual, 2^{nd},ed. Cold Spring Harbor Laboratory*, Cold Spring Harbor Laboratory Press, Cold Spring Harbor, NY, 1989), Davis et al., *Basic Methods in Molecular Biology* (1986) and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include, but are not limited to, those which confer resistance to drugs or which complement a defect in the host cell. They include e. g. versatile marker genes that can be used for transformation of most filamentous fungi and yeasts such as acetamidase genes or cDNAs (the *amdS, niaD, facA* genes or cDNAs from *A. nidulans, A. oryzae* or *A. niger*), or genes providing resistance to antibiotics like G418, hygromycin, bleomycin, kanamycin, methotrexate, phleomycin orbenomyl resistance (benA). Alternatively, specific selection markers can be used such as auxotrophic markers which require corresponding mutant host strains: e. g.URA3 (from S. *cerevisiae* or analogous genes from other yeasts), *pyrG* or *pyrA* (from *A. nidulans* or *A. niger*), *argB* (from *A. nidulans* or *A. niger*) or *trpC.* In a preferred embodiment the selection marker is deleted from the transformed host cell after introduction of the expression construct so as to obtain transformed host cells capable of producing the polypeptide which are free of selection marker genes.

Other markers include ATP synthetase, subunit 9 (*oliC*), orotidine-5'-phosphatedecarboxylase (*pvrA*), the bacterial G418 resistance gene (this may also be used in yeast, but not in fungi), the ampicillin resistance gene (*E*. *coli*)*,* the neomycin resistance gene (*Bacillus*) and the *E. coli uidA* gene, coding for β-glucuronidase (GUS). Vectors may be used *in vitro,* for example for the production of RNA or used to transfect or transform a host cell.

Expression of proteins in prokaryotes is often carried out in *E. coli* with vectors containing constitutive or inducible promoters directing the expression of either fusion or non-fusion proteins. Fusion vectors add a number of amino acids to a protein encoded therein, e.g. to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein.

As indicated, the expression vectors will preferably contain selectable markers. Such markers include dihydrofolate reductase or neomycin resistance for eukaryotic cell culture and tetracyline or ampicillin resistance for culturing in *E. coli* and other bacteria. Representative examples of appropriate host include bacterial cells, such as *E. coli, Streptomyces Salmonella typhimurium* and certain *Bacillus* species; fungal cells such as *Aspergillus* species, for example *A. niger*, *A. oryzae* and *A. nidulans,* such as yeast such as *Kluyveromyces*, for example *K*. *lactis* and/or *Puchia*, for example *P. pastoris*; insect cells such as *Drosophila* S2 and *Spodoptera* Sf9; animal cells such as CHO, COS and *Bowes melanoma*; and plant cells. Appropriate culture mediums and conditions for the above-described host cells are known in the art.

Vectors preferred for use in bacteria are for example disclosed in WO-A1-2004/074468. Other suitable vectors will be readily apparent to the skilled artisan.

Known bacterial promotors suitable for use in the present invention include the promoters disclosed in WO-A1-2004/074468.

Transcription of the DNA encoding the polypeptides of the present invention by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretation signal may be incorporated into the expressed polypeptide. The signals may be endogenous to the polypeptide or they may be heterologous signals.

The ZFX polypeptide may be expressed in a modified form, such as a fusion protein, and may include not only secretion signals but also additional heterologous functional regions. Thus, for instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification.

The invention provides an isolated polypeptide having the amino acid sequence according to SEQ ID NO: 3, and an amino acid sequence obtainable by expressing the polynucleotide of SEQ ID NO: 1 or 2 in an appropriate host. Also, a peptide or polypeptide comprising a functional equivalent of the above polypeptides is comprised within the present invention. The above polypeptides are collectively comprised in the term "polypeptides according to the invention"

The terms "peptide" and "oligopeptide" are considered synonymous (as is commonly recognized) and each term can be used interchangeably as the context requires to indicate a chain of at least two amino acids coupled by peptidyl linkages. The word "polypeptide" is used herein for chains containing more than seven amino acid residues. All oligopeptide and polypeptide formulas or sequences herein are written from left to right and in the direction from amino terminus to carboxy terminus. The one-letter code of amino acids used herein is commonly known in the art and can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989)

By "isolated" polypeptide or protein is intended a polypeptide or protein removed from its native environment. For example, recombinantly produced polypeptides and proteins expressed in host cells are considered isolated for the purpose of the invention as are native or recombinant polypeptides which have been substantially purified by any suitable technique such as, for example, the single-step purification method disclosed in Smith and Johnson, Gene 67:31-40 (1988).

The ZFX proline-specific protease according to the invention can be recovered and purified from recombinant cell cultures by methods known in the art. Most preferably, high performance liquid chromatography ("HPLC") is employed for purification.

Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

The invention also features biologically active fragments of the polypeptides according to the invention.

Biologically active fragments of a polypeptide of the invention include polypeptides comprising amino acid sequences sufficiently identical to or derived from the amino acid sequence of the ZFX protein (e.g., the amino acid sequence of SEQ ID NO: 3), which include fewer amino acids than the full length protein but which exhibit at least one biological activity of the corresponding full-length protein. Typically, biologically active fragments comprise a domain or motif with at least one activity of the ZFX protein. A biologically active fragment of a protein of the invention can be a polypeptide which is, for example, 10, 25, 50, 100 or more amino acids in length. Moreover, other biologically active portions, in which other regions of the protein are deleted, can be prepared by recombinant techniques and evaluated for one or more of the biological activities of the native form of a polypeptide of the invention.

The invention also features nucleic acid fragments which encode the above biologically active fragments of the ZFX protein.

The proteins of the present invention or functional equivalents thereof, e.g., biologically active portions thereof, can be operatively linked to a non-ZFX polypeptide (e.g., heterologous amino acid sequences) to form fusion proteins. A "non-ZFX polypeptide" refers to a polypeptide having an amino acid sequence corresponding to a protein which is not substantially homologous to the ZFX protein. Such "non-ZFX polypeptide" can be derived from the same or a different organism. Within a ZFX fusion protein the ZFX polypeptide can correspond to all or a biologically active fragment of a ZFX protein. In a preferred embodiment, a ZFX fusion protein comprises at least two biologically active portions of a ZFX protein. Within the fusion protein, the term "operatively linked" is intended to indicate that the ZFX polypeptide and the non-ZFX polypeptide are fused in-frame to each other. The non-ZFX polypeptide can be fused to the N-terminus or C-terminus of the ZFX polypeptide.

For example, in one embodiment, the fusion protein is a GST-ZFX fusion protein in which the ZFX sequences are fused to the C-terminus of the GST sequences. Such fusion proteins can facilitate the purification of recombinant ZFX. In another embodiment, the fusion protein is a ZFX protein containing a heterologous signal sequence at its N-terminus. In certain host cells (e.g., mammalian and yeast host cells), expression and/or secretion of ZFX can be increased through use of a hetereologous signal sequence.

In another example, the gp67 secretory sequence of the baculovirus envelope protein can be used as a heterologous signal sequence (Current Protocols in Molecular Biology, Ausubel et al., eds., John Wiley & Sons, 1992). Other examples of eukaryotic heterologous signal sequences include the secretory sequences of melittin and human placental alkaline phosphatase (Stratagene; La Jolla, California). In yet another example, useful prokarytic heterologous signal sequences include the *pho*A secretory signal (Sambrook *et al*., *supra*) and the protein A secretory signal (Pharmacia Biotech; Piscataway, New Jersey).

A signal sequence can be used to facilitate secretion and isolation of a protein or polypeptide of the invention. Signal sequences are typically characterized by a core of hydrophobic amino acids, which are generally cleaved from the mature protein during secretion in one or more cleavage events. Such signal peptides contain processing sites that allow cleavage of the signal sequence from the mature proteins as they pass through the secretory pathway. The signal sequence directs secretion of the protein, such as from a eukaryotic host into which the expression vector is transformed, and the signal sequence is subsequently or concurrently cleaved. The protein can then be readily purified from the extracellular medium by known methods. Alternatively, the signal sequence can be linked to the protein of interest using a sequence, which facilitates purification, such as with a GST domain. Thus, for instance, the sequence encoding the polypeptide may be fused to a marker sequence, such as a sequence encoding a peptide, which facilitates purification of the fused polypeptide. In certain preferred embodiments of this aspect of the invention, the marker sequence is a hexa-histidine peptide, such as the tag provided in a pQE vector (Qiagen, Inc.), among others, many of which are commercially available. As described in Gentz et al, Proc. Natl. Acad. Sci. USA 86:821-824 (1989), for instance, hexa-histidine provides for convenient purification of the fusion protein. The HA tag is another peptide useful for purification which corresponds to an epitope derived of influenza hemaglutinin protein, which has been described by Wilson et al., Cell 37:767 (1984), for instance.

Secreted enzymes, like the amino acid sequence of SEQ ID NO: 3, are often synthesized including a pre- or signal-sequence and / or a pro-sequence. These sequences are often removed from the protein either during or after the secretion process. The mature secreted protein therefore often does not contain these pre- and pro-sequences anymore. A processed version of SEQ ID NO: 3 lacking possible pre- or pro-sequences is part of the invention. A possible processing site in the amino acid sequence of SEQ ID NO: 3 is located at the carboxy-terminus of amino acid 20. The mature enzyme according to the invention will in this case start at amino acid number 21. All other modifications from the amino acid sequence of SEQ ID NO: 3 due to further processing are allowed as long that they do not disturb the activity of the enzyme.

Preferably, a ZFX fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). A ZFX-encoding nucleic acid can be cloned into such an expression vector such that the fusion moiety is linked in-frame to the ZFX protein.

The terms "variants", "functional variants" and "functional equivalents" are used interchangeably herein. Variants/functional equivalents of ZFX polynucleotides are isolated nucleic acid fragments that encode a polypeptide that exhibits a particular function of the ZFX *Penicillium chrysogenum* proline-specific protease as defined herein. A functional equivalent of a ZFX polypeptide according to the invention is a polypeptide that exhibits at least one function of a *Penicillium chrysogenum* proline-specific protease as defined herein. Functional equivalents therefore also encompass biologically active fragments.

A polypeptide of the invention may thus comprise the amino acid sequence set forth in SEQ ID NO: 3 or a substantially homologous sequence, or a fragment of either sequence having proline-specific protease activity. In general, the naturally occurring amino acid sequence shown in SEQ ID NO: 3 is preferred.

The polypeptide of the invention may also comprise a naturally occurring variant or species homologue of the polypeptide of SEQ ID NO: 3.

A variant is a polypeptide that occurs naturally in, for example, fungal, bacterial, yeast or plant cells, the variant having proline-specific protease activity and a sequence substantially similar to the protein of SEQ ID NO: 3. The term "variants" refers to polypeptides which have the same essential character or basic biological functionality as the proline-specific protease of SEQ ID NO: 3, and includes allelic variants. Preferably, a variant polypeptide has at least the same level of proline-specific protease activity as the polypeptide of SEQ ID NO: 3. Variants include allelic variants either from the same strain as the polypeptide of SEQ ID NO: 3 or from a different strain of the same genus or species.

Similarly, a species homologue of the inventive protein is an equivalent protein of similar sequence which is a proline-specific protease and occurs naturally in another species.

Variants and species homologues can be isolated using the procedures described herein and performing such procedures on a suitable cell source, for example a bacterial, yeast, fungal or plant cell. Also possible is to use a probe of the invention to probe DNA libraries made from yeast, bacterial, fungal or plant cells in order to obtain clones expressing variants or species homologues of the polypeptide of SEQ ID NO:3. The methods that can be used to isolate variants and species homologues of a known gene are extensively described in literature, and known to those skilled in the art. These genes can be manipulated by conventional techniques to generate a polypeptide of the invention which thereafter may be produced by recombinant or synthetic techniques known *per* se.

Functional protein or polypeptide equivalents may contain only conservative substitutions of one or more amino acids of SEQ ID NO: 3 or substitutions, insertions or deletions of non-essential amino acids. Accordingly, a non-essential amino acid is a residue that can be altered in SEQ ID NO: 3 without substantially altering the biological function.

That is to say, the sequence of the polypeptide of SEQ ID NO: 3 and of variants and species homologues can also be modified to provide polypeptides of the invention. Amino acid substitutions may be made, for example from 1, 2 or 3 to 10, 20 or 30 substitutions. The same number of deletions and insertions may also be made. These changes may typically be made outside regions critical to the function of the polypeptide, as such a modified polypeptide will retain its proline-specific protease activity.

The term "conservative substitution" is intended to indicate a substitution in which the amino acid residue is replaced with an amino acid residue having a similar side chain. These families are known in the art and include amino acids with basic side chains (e.g. lysine, arginine and hystidine), acidic side chains (e.g. aspartic acid, glutamic acid), uncharged polar side chains (e.g., glycine, asparagines, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine tryptophan, histidine).

Polypeptides of the invention include fragments of the above mentioned full length polypeptides and of variants thereof, including fragments of the sequence set out in SEQ ID NO: 3. Such fragments will typically retain activity as an proline-specific protease. Fragments may be at least 50, 100 or 200 amino acids long or may be this number of amino acids short of the full length sequence shown in SEQ ID NO: 3.

Polypeptides of the invention can, if necessary, be produced by synthetic means although usually they will be made recombinantly as described above. Synthetic and recombinant polypeptides may be modified, for example, by the addition of histidine residues or a T7 tag to assist their identification or purification, or by the addition of a signal sequence to promote their secretion from a cell.

Thus, the variant sequences may comprise those derived from strains of *Penicillium* other than the strain from which the polypeptide of SEQ ID NO:3 was isolated. Variants can be identified from other *Penicillium* strains by looking for proline-specific protease activity and cloning and sequencing as described herein. Variants may include the deletion, modification or addition of single amino acids or groups of amino acids within the protein sequence, as long as the peptide maintains the basic biological functionality of the proline-specific protease of SEQ ID NO: 3

Amino acid substitutions may be made, for example from 1, 2 or from 3 to 10, 20 or 30 substitutions. The modified polypeptide will generally retain activity as a proline-specific protease. Conservative amino acid substitutions may be made; such substitutions are well known in the art.

Shorter or longer polypeptide sequences are within the scope of the invention. For example, a peptide of at least 50 amino acids or up 100, 150, 200, 300, 400, 500, 600, 700 or 800 amino acids in length is considered to fall within the scope of the invention as long as it demonstrates the basic biological functionality of the proline-specific protease of SEQ ID NO:3. In particular, but not exclusively, this aspect of the invention encompasses the situation in which the protein is a fragment of the complete protein sequence.

For the present invention it is preferable that the protein of interest is actively secreted into the growth medium. Secreted proteins are normally originally synthesized as pre-proteins and the pre-sequence (signal sequence) is subsequently removed during the secretion process. The secretion process is basically similar in prokaryotes and eukaryotes: the actively secreted pre-protein is threaded through a membrane, the signal sequence is removed by a specific signal peptidase, and the mature protein is (re)-folded. Also for the signal sequence a general structure can be recognized. Signal sequences for secretion are located at the amino-terminus of the pre-protein, and are generally 15-35 amino-acids in length. The amino-terminus preferably contains positively charged amino-acids, and preferably no acidic amino-acids. It is thought that this positively charged region interacts with the negatively charged head groups of the phospholipids of the membrane. This region is followed by a hydrophobic, membrane-spanning core region. This region is generally 10-20 amino-acids in length and consists mainly of hydrophobic amino-acids. Charged amino-acids are normally not present in this region. The membrane spanning region is followed by the recognition site for signal peptidase. The recognition site consists of amino-acids with the preference for small-X-small. Small amino-acids can be alanine, glycine, serine or cysteine. X can be any amino acids.

Functional nucleic acid equivalents may typically contain silent mutations or mutations that do not alter the biological function of encoded polypeptide. Accordingly, the invention provides nucleic acid molecules encoding ZFX proteins that contain changes in amino acid residues that are not essential for a particular biological activity. Such ZFX proteins differ in amino acid sequence from SEQ ID NO: 3 yet retain at least one biological activity thereof. In one embodiment the isolated nucleic acid molecule comprises a nucleotide sequence encoding a protein, wherein the protein comprises a substantially homologous amino acid sequence of at least about 60%, 65%, 70%, 75%, 80%, 85%, 90%, 95%, 96%, 97%, 98%, 99% or more homologous to the amino acid sequence shown in SEQ ID NO: 3.

For example, guidance concerning how to make phenotypically silent amino acid substitutions is provided in Bowie, J.U. et al., Science 247:1306-1310 (1990) and the references cited therein. As the authors state, these studies have revealed that proteins are surprisingly tolerant of amino acid substitutions. The authors further indicate which changes are likely to be permissive at a certain position of the protein.

An isolated nucleic acid molecule encoding a ZFX protein homologous to the protein according to SEQ ID NO: 3 can be created by introducing one or more nucleotide substitutions, additions or deletions into the coding nucleotide sequences according to SEQ ID NO: 1 or 2 such that one or more amino acid substitutions, deletions or insertions are introduced into the encoded protein. Such mutations may be introduced by standard techniques, such as site-directed mutagenesis and PCR-mediated mutagenesis.

The term "functional equivalents" also encompasses orthologues of the *Penicillium chrysogenum* proline-specific protease protein. Orthologues of the *Penicillium chrysogenum* ZFX protein are proteins that can be isolated from other strains or species and possess a similar or identical biological activity. Such orthologues can readily be identified as comprising an amino acid sequence that is substantially homologous to SEQ ID NO: 3.

As defined herein, the term "substantially homologous" refers to a first amino acid or nucleotide sequence which contains a sufficient or minimum number of identical or equivalent (e.g., with similar side chain) amino acids or nucleotides to a second amino acid or nucleotide sequence such that the first and the second amino acid or nucleotide sequences have a common domain. For example, amino acid or nucleotide sequences which contain a common domain having about 60%, preferably 65%, more preferably 70%, even more preferably 75%, 80%, 85%, 90%, 95%, 96%, 97%, 98% or 99% identity or more are defined herein as sufficiently identical.

Also, nucleic acids encoding other ZFX family members, which thus have a nucleotide sequence that differs from SEQ ID NO: 1 or 2, are within the scope of the invention. Moreover, nucleic acids encoding ZFX proteins from different species which can have a nucleotide sequence which differs from SEQ ID NO: 1 or 2 are within the scope of the invention.

Nucleic acid molecules corresponding to variants (e.g. natural allelic variants) and homologues of the ZFX polynucleotide of the invention can be isolated based on their homology to the ZFX nucleic acids disclosed herein using the cDNAs disclosed herein or a suitable fragment thereof, as a hybridization probe according to standard hybridization techniques preferably under highly stringent hybridization conditions.

In addition to naturally occurring allelic variants of the ZFX sequence, the skilled person will recognise that changes can be introduced by mutation into the nucleotide sequences of SEQ ID NO: 1 or SEQ ID NO: 2 thereby leading to changes in the amino acid sequence of the ZFX protein without substantially altering the function of the ZFX protein.

In another aspect of the invention, improved ZFX proteins are provided. Improved ZFX proteins are proteins wherein at least one biological activity is improved. Such proteins may be obtained by randomly introducing mutations along all or part of the ZFX coding sequence, such as by saturation mutagenesis, and the resulting mutants can be expressed recombinantly and screened for biological activity. For instance, the art provides for standard assays for measuring the enzymatic activity of proline-specific proteases and thus improved proteins may easily be selected.

In a preferred embodiment the ZFX protein has an amino acid sequence according to SEQ ID NO: 3. In another embodiment, the ZFX polypeptide is substantially homologous to the amino acid sequence according to SEQ ID NO: 3 and, typically, retains at least one biological activity of a polypeptide according to SEQ ID NO: 3, yet differs in amino acid sequence due to natural variation or mutagenesis as described above.

In a further preferred embodiment, the ZFX protein has an amino acid sequence encoded by an isolated nucleic acid fragment capable of hybridizing to a nucleic acid according to SEQ ID NO: 1 or 2, preferably under highly stringent hybridization conditions.

Accordingly, the ZFX protein is preferably a protein which comprises an amino acid sequence at least about 60%, 65%, 70%, 75%, 80%, 85%, 90%, 95%, 96%, 97%, 98%, 99% or more homologous to (i.e. shares sequence identity with) the amino acid sequence shown in SEQ ID NO: 3. Typically, such a protein retains at least one functional activity of the polypeptide according to SEQ ID NO: 3, for example in relation to proline-specific protease activity.

Functional equivalents of a protein according to the invention can also be identified e.g. by screening combinatorial libraries of mutants, e.g. truncation mutants, of the protein of the invention for proline-specific protease activity. In one embodiment, a variegated library of variants is generated by combinatorial mutagenesis at the nucleic acid level. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g. for phage display). There are a variety of methods that can be used to produce libraries of potential variants of the polypeptides of the invention from a degenerate oligonucleotide sequence. Methods for synthesizing degenerate oligonucleotides are known in the art (see, e.g., Narang (1983) Tetrahedron 39:3; Itakura *et al.* (1984) Annu. Rev. Biochem. 53:323; Itakura *et al.* (1984) Science 198:1056; Ike *et al.* (1983) Nucleic Acid Res. 11:477).

In addition, libraries of fragments of the coding sequence of a polypeptide of the invention can be used to generate a variegated population of polypeptides for screening a subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations of truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of a protein of the invention (Arkin and Yourvan (1992) Proc. Natl. Acad. Sci. USA 89:7811-7815; Delgrave et al. (1993) Protein Engineering 6(3): 327-331).

It is to be understood that skilled persons may, using routine techniques, make nucleotide substitutions that do not affect the polypeptide sequence encoded by the poly-nucleotides of the invention to reflect the codon usage of any particular host organism in which the polypeptides of the invention are to be expressed.

The coding sequence of SEQ ID NO: 1 and/or SEQ ID NO: 2 may be modified by nucleotide substitutions, for example from 1, 2 or 3 to 10, 25, 50, 100, or more substitutions. The polynucleotide of SEQ ID NO: 1 and/or SEQ ID NO: 2 may alternatively or additionally be modified by one or more insertions and/or deletions and/or by an extension at either or both ends. The modified polynucleotide generally encodes a polypeptide which has proline-specific protease activity. Degenerate substitutions may be made and/or substitutions may be made which would result in a conservative amino acid substitution when the modified sequence is translated, for example as discussed with reference to polypeptides later.

The sequences provided by the present invention may also be used as starting materials for the construction of "second generation" enzymes. "Second generation" proline-specific proteases are proline-specific proteases altered by mutagenesis techniques (e.g. site-directed mutagenesis or gene shuffling techniques), which have properties that differ from those of wild-type proline-specific protease or recombinant proline-specific protease such as those produced by the present invention. For example, their temperature or pH optimum, specific activity, substrate affinity or thermostability may be altered so as to be better suited for use in a particular process.

Amino acids essential to the activity of the proline-specific protease of the invention, and therefore preferably subject to substitution, may be identified according to procedures known in the art, such as site-directed mutagenesis or alanine-scanning mutagenesis. In the latter technique mutations are introduced at every residue in the molecule, and the resultant mutant molecules are tested for biological activity (e.g. proline-specific protease activity) to identify amino acid residues that are critical to the activity of the molecule. Sites of enzyme-substrate interaction can also be determined by analysis of crystal structure as determined by such techniques as nuclear magnetic resonance, crystallography or photo-affinity labelling.

Gene shuffling techniques provide a random way to introduce mutations in a polynucleotide sequence. After expression the isolates with the best properties are re-isolated, combined and shuffled again to increase the genetic diversity. By repeating this procedure a number of times, genes that code for vastly improved proteins can be isolated. Preferably the gene shuffling procedure is started with a family of genes that code for proteins with a similar function. The family of polynucleotide sequences provided with this invention would be well suited for gene shuffling to improve the properties of secreted proline-specific proteases.

Alternatively classical random mutagenesis techniques and selection, such as mutagenesis with NTG treatment or UV mutagenesis, can be used to improve the properties of a protein. Mutagenesis can be performed directly on isolated DNA, or on cells transformed with the DNA of interest. Alternatively, mutations can be introduced in isolated DNA by a number of techniques that are known to the person skilled in the art. Examples of these methods are error-prone PCR, amplification of plasmid DNA in a repair-deficient host cell, etc.

In addition to the ZFX gene sequence shown in SEQ ID NO: 1 or 2, it will be apparent for the person skilled in the art that DNA sequence polymorphisms may exist within a given population, which may lead to changes in the amino acid sequence of the ZFX protein. Such genetic polymorphisms may exist in cells from different populations or within a population due to natural allelic variation. Allelic variants may also include functional equivalents.

Fragments of a polynucleotide according to the invention may also comprise polynucleotides not encoding functional polypeptides. Such polynucleotides may function as probes or primers for a PCR reaction.

Nucleic acids according to the invention irrespective of whether they encode functional or non-functional polypeptides can be used as hybridization probes or polymerase chain reaction (PCR) primers. Uses of the nucleic acid molecules of the present invention that do not encode a polypeptide having a ZFX activity include, inter alia, (1) isolating the gene encoding the ZFX protein, or allelic variants thereof from a cDNA library e.g. from other organisms than *Penicillium chrysogenum*; (2) *in situ* hybridization (e.g. FISH) to metaphase chromosomal spreads to provide precise chromosomal location of the ZFX gene as described in Verma et al., Human Chromosomes: a Manual of Basic Techniques, Pergamon Press, New York (1988); (3) Northern blot analysis for detecting expression of ZFX mRNA in specific tissues and/or cells and 4) probes and primers that can be used as a diagnostic tool to analyse the presence of a nucleic acid hybridizable to the ZFX probe in a given biological (e.g. tissue) sample.

Also encompassed by the invention is a method of obtaining a functional equivalent of a ZFX gene. Such a method entails obtaining a labelled probe that includes an isolated nucleic acid which encodes all or a portion of the protein sequence according to SEQ ID NO: 3 or a variant thereof; screening a nucleic acid fragment library with the labelled probe under conditions that allow hybridization of the probe to nucleic acid fragments in the library, thereby forming nucleic acid duplexes, and preparing a full-length gene sequence from the nucleic acid fragments in any labelled duplex to obtain a gene related to the ZFX gene.

In one embodiment, a ZFX nucleic acid of the invention is at least 60%, 65%, 70%, 75%, 80%, 85%, 90%, 91%, 92%, 93%, 94%, 95%, 96%, 97%, 98%, 99%, or more homologous to a nucleic acid sequence shown in SEQ ID NO: 1 or SEQ ID NO: 2 or the complement of either thereof.

In another embodiment, the invention features cells, e.g., transformed host cells or recombinant host cells that contain a nucleic acid encompassed by the invention. A "transformed cell" or "recombinant cell" is a cell into which (or into an ancestor of which) has been introduced, by means of recombinant DNA techniques, a nucleic acid according to the invention. Both prokaryotic and eukaryotic cells are included, e.g., bacteria, fungi, yeast, and the like, especially preferred are cells from filamentous fungi, in particular *Aspergillus niger.*

Accordingly, the invention provides a host cell comprising a polypeptide, a polynucleotide or a vector of the invention. The polynucleotide may be heterologous to the genome of the host cell. The term "heterologous", usually with respect to the host cell, means that the polynucleotide does not naturally occur in the genome of the host cell or that the polypeptide is not naturally produced by that cell.

A host cell can be chosen that modulates the expression of the inserted sequences, or modifies and processes the gene product in a specific, desired fashion. Such modifications (e.g., glycosylation) and processing (e.g., cleavage) of protein products may facilitate optimal functioning of the protein.

Various host cells have characteristic and specific mechanisms for post-translational processing and modification of proteins and gene products. Appropriate cell lines or host systems familiar to those of skill in the art of molecular biology and/or microbiology can be chosen to ensure the desired and correct modification and processing of the foreign protein expressed. To this end, eukaryotic host cells that possess the cellular machinery for proper processing of the primary transcript, glycosylation, and phosphorylation of the gene product can be used. Such host cells are well known in the art.

If desired, a cell as described above may be used to in the preparation of a polypeptide according to the invention. Such a method typically comprises cultivating a host cell (e.g. transformed or transfected with an expression vector as described above) under conditions to provide for expression (by the vector) of a coding sequence encoding the polypeptide, and optionally recovering the expressed polypeptide.

Polynucleotides of the invention can be incorporated into a recombinant replicable vector, e.g. an expression vector. The vector may be used to replicate the nucleic acid in a compatible host cell.

Thus in a further embodiment, the invention provides a method of making a polynucleotide of the invention by introducing a polynucleotide of the invention into a replicable vector, introducing the vector into a compatible host cell, and growing the host cell under conditions which bring about the replication of the vector. The vector may be recovered from the host cell.

Suitable host cells include bacteria such as *E. coli*, yeast, mammalian cell lines and other eukaryotic cell lines, for example insect cells such as Sf9 cells and (e.g. filamentous) fungal cells.

Preferably the polypeptide is produced as a secreted protein in which case the nucleotide sequence encoding a mature form of the polypeptide in the expression construct is operably linked to a nucleotide sequence encoding a signal sequence. Preferably the signal sequence is native (homologous) to the nucleotide sequence encoding the polypeptide. Alternatively the signal sequence is foreign (heterologous) to the nucleotide sequence encoding the polypeptide, in which case the signal sequence is preferably endogenous to the host cell in which the nucleotide sequence according to the invention is expressed. Examples of suitable signal sequences for yeast host cells are the signal sequences derived from yeast a-factor genes. Similarly, a suitable signal sequence for filamentous fungal host cells is e. g. a signal sequence derived from a filamentous fungal amyloglucosidase (AG) gene, e. g. the *A. niger gla*A gene. This may be used in combination with the amyloglucosidase (also called (gluco) amylase) promoter itself, as well as in combination with other promoters. Hybrid signal sequences may also be used with the context of the present invention.

Preferred heterologous secretion leader sequences are those originating from the fungal amyloglucosidase (AG) gene (g/aA-both 18 and 24 amino acid versions e. g. from *Aspergillus*), the α-factor gene (yeasts e. g. *Saccharomyces* and *Kluyveromyces*) or the α-amylase gene (*Bacillus*).

The vectors may be transformed or transfected into a suitable host cell as described above to provide for expression of a polypeptide of the invention. This process may comprise culturing a host cell transformed with a vector, such as an expression vector, as described above under conditions to provide for expression by the vector of a coding sequence encoding the polypeptide and, optionally, recovering the expressed polypeptide.

The invention encompasses a polypeptide obtainable or obtained by such a process.

In a preferred embodiment a polypeptide of the invention is produced from a fungus, more preferably from an *Aspergillus,* most preferably from *Aspergillus niger.*

The invention thus provides host cells transformed or transfected with or comprising a polynucleotide or vector of the invention. Preferably the polynucleotide is carried in a vector for the replication and expression of the polynucleotide. The cells will be chosen to be compatible with the said vector and may for example be prokaryotic (for example bacterial), fungal, yeast or plant cells.

Suitable host cells are preferably prokaryotic microorganisms such as bacteria, or more preferably eukaryotic organisms, for example fungi, such as yeasts or filamentous fungi, or plant cells. In general, yeast cells are preferred over fungal cells because they are easier to manipulate. However, some proteins are either poorly secreted from yeasts, or in some cases are not processed properly (e. g. hyperglycosylation in yeast). In these instances, a fungal host organism should be selected.

The host cell may over-express the polypeptide, and techniques for engineering over-expression are well known. The host may thus have two or more copies of the encoding polynucleotide (and the vector may thus have two or more copies accordingly).

Bacteria from the genus *Bacillus* are very suitable as heterologous hosts because of their capability to secrete proteins into the culture medium. Other bacteria suitable as hosts are those from the genera *Streptomyces* and *Pseudomonas.* A preferred yeast host cell for the expression of the DNA sequence encoding the polypeptide is of the genera *Saccharomyces*, *Kluyveromyces*, *Hansenula*, *Pichia*, *Yarrowia*, and *Schizosaccharomyces.*

More preferably a yeast host cell is selected from the group consisting of the species *Saccharomyces cerevisiae, Kluyveromyces lactis* (also known as *Kluyveromycesmarxianus* var.*lactis*), *Hansenulapolymorpha*, *Pichia pastoris*, *Yarrowia lipolytica* and *Schizosaccharomyces pombe.*

Most preferred are, however, (e. g. filamentous) fungal host cells. Preferred filamentous fungal host cells are selected from the group consisting of the genera *Aspergillus, Trichodernza, Fusarium, Disporotrichum, Penicillium, Acremonium, Neurospora*, *Thermoascus*, *Myceliophtora*, *Sporotrichum*, *Thielavia* and *Talaromyces.*

More preferably a filamentous fungal host cell is of the species *Aspergillus oryzae*, *Aspergillus sojae*, *Aspergillus nidulans*, or a species from the *Aspergillus niger* Group. These include, but are not limited to *Aspergillus niger*, *Aspergillusawamori*, *Aspergillus tubingensis, Aspergillus aculeatus, Aspergillus foetidus, Aspergillus nidulans*, *Aspergillus japonicus*, *Aspergillus oryzae* and *Aspergillus ficuum* and further consisting of the species *Trichoderma reesei*, *Fusarium graminearum*, *Penicillium chrysogenum*, *Acremonium alabamense*, *Neurospora crassa*, *Myceliophtora thernaophilurri*, *Sporotrichum cellulophilum*, *Disporotrichum dimorphosporum* and *Thielavia terrestris.*

Examples of preferred expression hosts within the scope of the present invention are fungi such as Aspergillus species (in particular those described in EP-A-184,438 and EP-A-284,603) and Trichoderma species; bacteria such as Bacillus species (in particular those described in EP-A-134,048 and EP-A-253,455), especially *Bacillus subtilis*, *Bacillus licheniformis*, *Bacillus amyloliquefaciens*, Pseudomonas species; and yeasts such as Kluyveromyces species (in particular those described in EP-A-096,430 such as *Kluyveromyces lactis* and in EP-A-301,670) and Saccharomyces species, such as *Saccharomyces cerevisiae.*

The invention encompasses processes for the production of the polypeptide of the invention by means of recombinant expression of a DNA sequence encoding the polypeptide. For this purpose the DNA sequence of the invention can be used for gene amplification and/or exchange of expression signals, such as promoters, secretion signal sequences, in order to allow economic production of the polypeptide in a suitable homologous or heterologous host cell. A homologous host cell is a host cell which is of the same species or which is a variant within the same species as the species from which the DNA sequence is derived. The term "heterologous", usually with respect to the host cell, means that the polynucleotide does not naturally occur in the genome of the host cell or that the polypeptide is not naturally produced by that cell.

Accordingly, the invention provides an improved means for producing the newly identified secreted proline-specific protease in high quantities and a relatively pure form ivia the overproduction of the *Penicillium* encoded enzyme using recombinant DNA techniques. A preferred way of doing this is via the overproduction of such a secreted proline-specific protease in a food grade host microorganism. Well known food grade microrganisms include *Aspergilli, Trichoderma, Streptomyces, Bacilli* and yeasts such as *Saccharomyces* and *Kluyveromyces.* An even more preferred way of doing this is via overproduction of the secreted *Penicillium* derived proline-specific protease in a food grade fungus such as *Aspergillus.* Most preferred is the over production of the secreted proline-specific protease in a food grade fungus in which the codon-usage of the proline-specific protease-encoding gene has been optimized for the food grade expression host used. In general, to enable the latter optimization routes, unique sequence information of a secreted proline-specific protease is desirable. More preferable the whole nucleotide sequence of the proline-specific protease encoding gene has to be available. Once the gene encoding a secreted proline-specific protease is transformed in a preferred host, selected strains can be used for fermentation and isolation of the secreted proline-specific protease protein from the fermentation broth.

Host cells according to the invention include plant cells, and the invention therefore extends to transgenic organisms, such as plants and parts thereof, which contain one or more cells of the invention. The cells may heterologously express the polypeptide of the invention or may heterologously contain one or more of the polynucleotides of the invention. The transgenic (or genetically modified) plant may therefore have inserted (e. g. stably) into its genome a sequence encoding one or more of the polypeptides of the invention. The transformation of plant cells can be performed using known techniques, for example using a Ti or a Ri plasmid from *Agrobacterium tumefaciens.* The plasmid (or vector) may thus contain sequences necessary to infect a plant, and derivatives of the Ti and/or Ri plasmids may be employed.

Alternatively direct infection of a part of a plant, such as a leaf, root or stem can be effected. In this technique the plant to be infected can be wounded, for example by cutting the plant with a razor or puncturing the plant with a needle or rubbing the plant with an abrasive. The wound is then inoculated with the *Agrobacterium.* The plant or plant part can then be grown on a suitable culture medium and allowed to develop into a mature plant. Regeneration of transformed cells into genetically modified plants can be achieved by using known techniques, for example by selecting transformed shoots using an antibiotic and by sub-culturing the shoots on a medium containing the appropriate nutrients, plant hormones and the like.

The invention thus includes cells that have been modified to express the proline-specific protease or a variant thereof. Such cells include transient, or preferably stable higher eukaryotic cell lines, such as mammalian cells or insect cells, lower eukaryotic cells, such as yeast and (e. g. filamentous) fungal cells or prokaryotic cells such as bacterial cells.

The present invention also relates to methods for producing a mutant cell of a parent cell, which comprises disrupting or deleting the endogenous nucleic acid sequence encoding the polypeptide or a control sequence thereof, which results in the mutant cell producing less of the polypeptide than the parent cell.

The construction of strains which have reduced proline-specific protease activity may be conveniently accomplished by modification or inactivation of a nucleic acid sequence necessary for expression of the proline-specific protease in the cell. The nucleic acid sequence to be modified or inactivated may be, for example, a nucleic acid sequence encoding the polypeptide or a part thereof essential for exhibiting proline-specific protease activity, or the nucleic acid sequence may have a regulatory function required for the expression of the polypeptide from the coding sequence of the nucleic acid sequence. An example of such a regulatory or control sequence may be a promoter sequence or a functional part thereof, i.e., a part which is sufficient for affecting expression of the polypeptide. Other control sequences for possible modification include, but are not limited to, a leader sequence, a poly-adenylation sequence, a pro-peptide sequence, a signal sequence, and a termination sequence.

Modification or inactivation of the nucleic acid sequence may be performed by subjecting the cell to mutagenesis and selecting cells in which the proline-specific protease producing capability has been reduced or eliminated. The mutagenesis, which may be specific or random, may be performed, for example, by use of a suitable physical or chemical mutagenizing agent, by use of a suitable oligo-nucleotide, or by subjecting the DNA sequence to PCR mutagenesis. Furthermore, the mutagenesis may be performed by use of any combination of these mutagenic agents.

Examples of a physical or chemical mutagenic agent suitable for the present purpose include ultraviolet (UV) irradiation, hydroxylamine, N-methyl-N'-nitro-N-nitrosoguanidine (NTG), O-methyl hydroxylamine, nitrous acid, ethyl methane sulphonate (EMS), sodium bisulphite, formic acid, and nucleotide analogues.

When such agents are used, the mutagenesis is typically performed by incubating the cell to be mutated in the presence of the mutagenic agent of choice under suitable conditions, and selecting for cells exhibiting reduced or no expression of proline-specific protease activity.

Modification or inactivation of production of a polypeptide of the present invention may be accomplished by introduction, substitution, or removal of one or more nucleotides in the nucleic acid sequence encoding the polypeptide or a regulatory element required for the transcription or translation thereof. For example, nucleotides may be inserted or removed so as to result in the introduction of a stop codon, the removal of the start codon, or a change of the open reading frame. Such modification or inactivation may be accomplished by site-directed mutagenesis or PCR mutagenesis in accordance with methods known in the art.

Although, in principle, the modification may be performed in vivo, i.e., directly on the cell expressing the nucleic acid sequence to be modified, it is preferred that the modification be performed in vitro as exemplified below.

An example of a convenient way to inactivate or reduce production of the proline-specific protease by a host cell of choice is based on techniques of gene replacement or gene interruption. For example, in the gene interruption method, a nucleic acid sequence corresponding to the endogenous gene or gene fragment of interest is mutated in vitro to produce a defective nucleic acid sequence which is then transformed into the host cell to produce a defective gene. By homologous recombination, the defective nucleic acid sequence replaces the endogenous gene or gene fragment. Preferably the defective gene or gene fragment also encodes a marker which may be used to select for transformants in which the gene encoding the polypeptide has been modified or destroyed.

Alternatively, modification or inactivation of the nucleic acid sequence encoding a polypeptide of the present invention may be achieved by established anti-sense techniques using a nucleotide sequence complementary to the polypeptide encoding sequence. More specifically, production of the polypeptide by a cell may be reduced or eliminated by introducing a nucleotide sequence complementary to the nucleic acid sequence encoding the polypeptide. The anti-sense poly-nucleotide will then typically be transcribed in the cell and will be capable of hybridizing to the mRNA encoding the proline-specific protease. Under conditions allowing the complementary anti-sense nucleotide sequence to hybridize to the mRNA, the amount of the proline-specific protease produced in the cell will be reduced or eliminated.

It is preferred that the cell to be modified in accordance with the methods of the present invention is of microbial origin, for example, a fungal strain which is suitable for the production of desired protein products, either homologous or heterologous to the cell.

The present invention further relates to a mutant cell of a parent cell which comprises a disruption or deletion of the endogenous nucleic acid sequence encoding the polypeptide or a control sequence thereof, which results in the mutant cell producing less of the polypeptide than the parent cell.

The polypeptide-deficient mutant cells so created are particularly useful as host cells for the expression of homologous and/or heterologous polypeptides. Therefore, the present invention further relates to methods for producing a homologous or heterologous polypeptide comprising (a) culturing the mutant cell under conditions conducive for production of the polypeptide; and (b) recovering the polypeptide. In the present context, the term "heterologous polypeptides" is defined herein as polypeptides which are not native to the host cell, a native protein in which modifications have been made to alter the native sequence, or a native protein whose expression is quantitatively altered as a result of a manipulation of the host cell by recombinant DNA techniques.

In a still further aspect, the present invention provides a method for producing a protein product essentially free of proline-specific protease activity by fermentation of a cell which produces both a proline-specific protease poly-peptide of the present invention as well as the protein product of interest. The method comprises adding an effective amount of an agent capable of inhibiting proline-specific protease activity to the fermentation broth either during or after the fermentation has been completed, recovering the product of interest from the fermentation broth, and optionally subjecting the recovered product to further purification. Alternatively, after cultivation the resultant culture broth can be subjected to a pH or temperature treatment so as to reduce the proline-specific protease activity substantially, and allow recovery of the product from the culture broth. The combined pH or temperature treatment may be performed on a protein preparation recovered from the culture broth.

The method described above for the production an essentially proline-specific protease-free product is of particular interest in the production of eukaryotic polypeptides, in particular in the production of fungal proteins such as enzymes. The proline-specific protease-deficient cells may also be used to express heterologous proteins of interest for the food industry, or of pharmaceutical interest.

According to the present invention, the production of the polypeptide of the invention can be effected by the culturing of microbial expression hosts, which have been transformed with one or more polynucleotides of the present invention, in a conventional nutrient fermentation medium.

The recombinant host cells according to the invention may be cultured using procedures known in the art. For each combination of a promoter and a host cell, culture conditions are available which are conducive to the expression the DNA sequence encoding the polypeptide. After reaching the desired cell density or titre of the polypeptide the culture is stopped and the polypeptide is recovered using known procedures.

The fermentation medium can comprise a known culture medium containing a carbon source (e. g. glucose, maltose, molasses, etc.), a nitrogen source (e. g. ammonium sulphate, ammonium nitrate, ammonium chloride, etc.), an organic nitrogen source (e. g. yeast extract, malt extract, peptone, etc.) and inorganic nutrient sources (e.g. phosphate, magnesium, potassium, zinc, iron, etc.). Optionally, an inducer (e. g. cellulose, pectin, maltose, maltodextrin or xylogalacturonan) may be included.

The selection of the appropriate medium may be based on the choice of expression host and/or based on the regulatory requirements of the expression construct. Such media are known to those skilled in the art. The medium may, if desired, contain additional components favouring the transformed expression hosts over other potentially contaminating microorganisms.

The fermentation can be performed over a period of 0.5-30 days. It may be a batch, continuous or fed-batch process, suitably at a temperature in the range of, for example, from about 0 to 45°C and/or at a pH, for example, from about 2 to about 10. Preferred fermentation conditions are a temperature in the range of from about 20 to about 37°C and/or at a pH of from about 3 to about 9. The appropriate conditions are usually selected based on the choice of the expression host and the protein to be expressed.

After fermentation, if necessary, the cells can be removed from the fermentation broth by means of centrifugation or filtration. After fermentation has stopped or after removal of the cells, the polypeptide of the invention may then be recovered and, if desired, purified and isolated by conventional means.

A polypeptide of the invention which has proline-specific protease activity may be in an isolated form. As defined herein, an isolated polypeptide may be an endogenously (classically) produced or a recombinant polypeptide which is essentially free from other non-proline-specific protease polypeptides, and is typically at least about 20% pure, preferably at least about 40% pure, more preferably at least about 60% pure, even more preferably at least about 80% pure, still more preferably about 90% pure, and most preferably about 95% pure, about 98% pure or about 99% pure as determined by SDS-PAGE.

The polypeptide may be isolated by filtration, followed by concentration (for example ultrafiltration) or any other technique known in the art for obtaining pure proteins from crude solutions. The resulting preparation may be spray-dried or maintained as a liquid preparation. It will be understood that the polypeptide may be mixed with carriers or diluents which do not interfere with the intended purpose of the polypeptide, and thus the polypeptide in this form will still be regarded as isolated. It will generally comprise the polypeptide in a preparation in which more than 20%, for example more than 30%, 40%, 50%, 80%, 90%, 95% or 99%, by weight of the proteins in the preparation is a polypeptide of the invention.

Polypeptides of the invention may be provided in a form such that they are outside their natural cellular environment. Thus, they may be substantially isolated or purified, as discussed above, or in a cell in which they do not occur in nature, for example a cell of other fungal species, animals, plants or bacteria.

Preferably, the polypeptide of the invention is obtainable from a microorganism which possesses a gene encoding an enzyme with proline-specific protease activity. More preferably polypeptide of the invention is secreted from a microorganism. Even more preferably the microorganism is fungal, and optimally is a filamentous fungus. Preferred donor organisms are thus of the genus *Penicillium,* such as those of the species *Penicillium chrysogenum.*

The present invention provides an isolated polypeptide having an amino acid sequence which has a degree of amino acid sequence identity to the polypeptide of SEQ ID NO: 3 (i.e. the polypeptide) of at least about 60%, such at least about 70%, such at least about 80%, preferably at least about 85%, more preferably at least about 90%, even more preferably at least about 95%, still more preferably at least about 98%, and most preferably at least about 99%, and which has proline-specific protease activity.

Preferably, an isolated or purified polypeptide according to the invention preferably has at least 10 units of proline specific protease activity per gram of proteinaceous material. These units should be measured using the synthetic peptide Z-Gly-Pro-pNA at 37°C and pH 5, as described in the Methods section.

Typically, a polypeptide of the invention will also have similar or improved characteristics as the polypeptide of SEQ ID NO: 3 with respect to temperature optima and/or thermolability. Thus, the polypeptide may have a temperature optimum of about 60°C or less, about 50°C or less, about 40°C or less, about 37°C or less or lower.

It may be possible to inactivate a polypeptide of the invention by heating the polypeptide to about 60°C for from about 10 minutes to about 30 minutes, such as for from about 15 to about 25 minutes, such as for about 20 minutes. Such inactivation may be carried out using a pasteurization process. Inactivation in this context implies that there is no or substantially no detectable residual enzymatic activity following the heat inactivation process.

Accordingly, any of the methods of uses described herein in which a polypeptide of the invention is used may include a step of inactivating the polypeptide. Such a step may be a heat activation step as described above. The result of such a step may that there is no or substantially no detectable residual enzymatic activity of the polypeptide.

Polypeptides of the invention may be chemically modified, e.g. post-translationally modified. For example, they may be glycosylated (one or more times) or comprise modified amino acid residues. They may also be modified by the addition of histidine residues to assist their purification or by the addition of a signal sequence to promote secretion from the cell. The polypeptide may have amino- or carboxyl-terminal extensions, such as an amino-terminal methionine residue, a small linker peptide of up to about 20-25 residues, or a small extension that facilitates purification, such as a poly-histidine tract, an antigenic epitope or a binding domain.

A polypeptide of the invention may be labeled with a revealing label. The revealing label may be any suitable label which allows the polypeptide to be detected. Suitable labels include radioisotopes, e.g. ¹²⁵I, ³⁵S, enzymes, antibodies, polynucleotides and linkers such as biotin.

The polypeptides may be modified to include non-naturally occurring amino acids or to increase the stability of the polypeptide. When the proteins or peptides are produced by synthetic means, such amino acids may be introduced during production. The proteins or peptides may also be modified following either synthetic or recombinant production.

The polypeptides of the invention may also be produced using D-amino acids. In such cases the amino acids will be linked in reverse sequence in the C to N orientation. This is conventional in the art for producing such proteins or peptides.

A number of side chain modifications are known in the art and may be made to the side chains of the proteins or peptides of the present invention. Such modifications include, for example, modifications of amino acids by reductive alkylation by reaction with an aldehyde followed by reduction with NaBH₄, amidination with methylacetimidate or acylation with acetic anhydride.

A polypeptide of the invention may be used in any method or application in which proline-specific protease enzymatic activity is required.

The invention thus provides a composition comprising a polypeptide of the invention. Such a composition may be used in a method or use of the invention

Auxiliary enzyme activity, such as additional protease activity, may be required in a method or use according to the invention. Thus, the invention provides a composition comprising: (i) a polypeptide according to the invention and; (ii) a polypeptide providing activity which is different from the polypeptide in (i), such as a protease which is different from the polypeptide in (i).

For example, an additional, different proline-specific protease may be used. Examples of such enzymes are widely found in animals and plants and have been reported in microorganisms: for example, proline-specific proteases have been identified in species of *Aspergillus* (EP 0 522 428), *Flavobacterium* (EP 0 967 285) and *Aeromonas* (J.Biochem.113, 790-796), *Xanthomonas* and *Bacteroides.*

Alternatively, additional proteases may be used. Preferably, such additional proteases have acid pH optima. Examples of such enzymes are disclosed in WO 03/102195 and also herein.

Accordingly, the invention provides a method for the preparation of a protein hydrolysate, which method comprises contacting a protein substrate with a polypeptide or composition according to the invention. Thus, the invention also provides the use of a polypeptide or composition of the invention in the preparation of a protein hydrolysate. The invention also relates to a protein hydrolysate obtainable or obtained by such a method or use.

Such a method may be of use in the creation of non-bitter hydrolysates from proteinaceous substrates with unusual amino acid compositions. Such unusual amino acid compositions may offer serious benefits in certain food applications. Examples are casein or wheat gluten or maize protein isolate with high levels of hydrophobic amino acid residues present. Using the method of the invention, non-bitter hydrolysates can be made available to be used in infant and clinical nutrition, in therapeutic diets as well as in consumer diets and sport nutrition.

An embodiment of the present invention provides an enzyme mixture comprising an isolated, purified proline-specific protease (i.e. polypeptide of the invention) for the high yield production of protein hydrolysates having substantially low bitterness and low allergenic properties without the concomitant production of substantial levels of free amino acids. This enzyme mixture is suitable for preparing hydrolysates of various protein fractions. In particular, a protein substrate, such as a milk protein, may be incubated with an isolated, purified proline-specific protease and a subtilisin to produce a protein hydrolysate enriched in peptide fragments having a carboxy terminal proline. The term "enriched" is intended to mean that at least 8% of the peptide fragments in the hydrolysate product of enzymatic cleavage possess a carboxy terminal proline residue.

Protein hydrolysates obtained by hydrolysing a protein may comprise peptides wherein the molar fraction of peptides (%) carrying a carboxy terminal proline is at least two times the molar fraction (%) of proline in the protein substrate used to produce the hydrolysate.

The average length of the peptides in the hydrolysates is in general from 3 to 9 amino acids.

Preferred hydrolysates prepared using a polypeptide of the invention are: a whey hydrolysate which comprises peptides wherein the molar fraction of peptides carrying a carboxy terminal proline is at least 8%, preferably at least 15%, more preferably from 30 to 70%, a casein hydrolysate which comprises peptides wherein the molar fraction of peptide carrying a carboxy terminal proline is at least 25%, preferably from 30 to 70%, and a soy hydrolysate which comprises peptides wherein the molar fraction of peptides carrying a carboxy terminal proline is at least 20%, preferably from 30 to 70%.

In relation to the hydrolysates described above, by peptides or peptide fragments it is meant peptides with molecular masses from 400 to 2000 Dalton. These peptides can be analysed using methods known to those skilled in the art (for example using LC/MC).

In general in the production of the protein hydrolysates of the invention the protein substrate is substantially hydrolysed, advantageously for at least 50%. Preferably at least 10% of the protein substrate is converted into peptides having molecular masses from 400 to 2000 Dalton. More preferably from 20 to 90% and even more preferably from 30 to 80% of the protein substrate is converted into such peptides.

In another embodiment of the invention, a protein substrate may be incubated with an enzyme mixture comprising an isolated, purified proline-specific protease, a serine protease or a metallo protease and a carboxypeptidase to produce a protein hydrolysate enriched in peptide fragments having a carboxy terminal proline.

The enzyme mixture of the invention is particularly suitable for use in the production of protein hydrolysates intended for flavoring and nutrient enhancement of sport drinks and juice-based beverages, as the resulting hydrolyzed peptide mixture combines a very low bitterness profile with excellent solubility under the prevailing acidic conditions of such beverages. The enzyme mixture of the invention is characterised in that it contains at least one protease for example a serine protease or a metallo endoprotease in conjunction with a proline-specific protease to provide a primary hydrolysate. More specifically, the invention relates to an isolated, purified proline-specific protease (i.e. a polypeptide of the invention) and a serine protease or metallo protease enzyme mixture capable of producing a protein hydrolysate comprising peptide fragments, wherein at least 8%, preferably at least 15%, more preferably from 30 to 70% of said peptide fragments have a carboxy terminal proline.

Substrates for hydrolysis by an enzyme mixture of the invention include whole milk, skimmed milk, acid casein, rennet casein, acid whey products or cheese whey products. Quite surprisingly the *Aspergillus* derived proline specific protease does not only cleave at the carboxy-terminal side of proline residues but also at the carboxy-terminal side of hydroxyproline residues which makes other, collagen based animal proteins such as gelatine as well as bones or fish-bones containing residual meat, interesting substrates for the enzyme. Moreover, vegetable substrates like wheat gluten, milled barley and protein fractions obtained from, for example, soy, rice or corn are suitable substrates. Milk protein hydrolysates produced according to the invention may be used with or without additional filtration or purification steps in various speciality foods such as hypoallergenic hydrolysates for infant nutrition, basic hydrolysates for enteral and dietetic nutrition, as well as protein concentrates for various forms of health food. Thus, protein hydrolysates of the invention may be used to produce foodstuffs having low antigenicity, such as infant formula. In addition, enzyme preparations according to the invention may be used to reduce bitterness in foods flavored by at least one protein hydrolysate, even when the protein hydrolysate is present in large amounts. For example, foods may comprise between 5% and 10% (w/v) of a protein hydrolysate and still have their bitterness reduced using an enzyme preparation of the invention.

Accordingly, the invention provides a food or feed product or a beverage which comprises a protein hydrolysate as described herein.

A polypeptide of the present invention provides an isolated, purified proline-specific protease with an acidic pH optimum alone or in a composition comprising one or more additional enzymes for the preparation of a protein hydrolysate for various food applications.

Such an isolated, purified proline-specific protease may preferably have at least 10 units of proline specific protease activity per gram of proteinaceous material. These units should be measured using the synthetic peptide Z-Gly-Pro-pNA at 37°C and pH 5 in case the pH optimum of the proline-specific protease is below pH 6, for example in case of *Aspergillus niger* proline specific endo protease or else the units should be measured at pH = 7, as specified in the Materials and Methods section.

This isolated, purified enzyme, alone or in an enzyme mixture, overcomes a number of disadvantages of enzyme mixtures previously known in the art. Most importantly, a polypeptide of the invention is key in the production of hydrolysates which combine a low allergenic potential, a high yield and a low bitterness profile. Moreover, the hydrolysates produced with the isolated, purified proline-specific protease or an enzyme mixture comprising this proline-specific protease are acid stable and contain very low levels of free amino acids, such that minimal off-tastes are generated during heating steps, such as spray drying or product sterilisation. Hydrolysates according to the invention will contain less than 900 micromoles of free amino acids per gram of dry powder, preferably less than 300 micromoles of free amino acids per gram of dry powder, more preferably less than 150 micromoles of free amino acids per gram of dry powder, and even more preferably less than 50 micromoles per gram of dry powder.

The enzyme mixture according to the invention is characterised in that it comprises another endoprotease such as a serine protease or a metallo endoprotease in conjunction with an isolated, purified proline-specific protease which work together to provide a primary protein hydrolysate.

Serine proteases represent a well known class of alkaline endoproteases and some of its most important representatives such as subtilisin (E.C. 3.4.21.62) and chymotrypsin (E.C. 3.4.21.1) prefer cleavage of the peptide chain at the carboxy terminal side of hydrophobic amino acids such as Tyr, Trp, Phe and Leu. The enzyme mixture of the invention may contain chymotrypsin and/or subtilisin. Subtilisin is produced by species of Bacillus, has a particularly broad substrate specificity and a broad, alkaline pH optimum. The enzyme is optimally active between 50°C and 60°C. The enzyme is cheaply available as a regular commercial product and is useful in the production of, for example, various milk hydrolysates. Chymotrypsin may be obtained from animal pancreases, has a somewhat narrower substrate specificity at slightly more alkaline pH values than subtilisin and is optimally active below 50 degrees C.

The class of metallo endoproteases is wide spread in bacteria, fungi and higher organisms. They can be separated into the neutral and acid metalloproteases. Of these two subclasses only the neutral proteases exhibit the desirable cleavage preference i.e. cleaving the peptide chain on the carboxy terminal side of hydrophobic amino acid residues such as Phe and Leu. Well known representatives of the category of the neutral metallo proteases are bacillolysin (E.C. 3.4.24.28) and thermolysin (E.C. 3.4.24.27) and either, or both of these, may be present in the enzyme mixture of the invention. Both enzymes are obtained from *Bacillus* species and exhibit maximum activity under neutral or slightly alkaline conditions. Less well known representatives of these neutral metallo endoproteases have been obtained from *Aspergillus* species. In those cases in which the proline specific protease is not used for its debittering effects but to aid in the hydrolysis of proline rich protein sequences, combinations with the class of the acid metalloproteases, as for example deuterolysine (EC 3.4.24.39) can be advantageous.

Aside from the preparation of such hydrolysates, applications that take advantage of the bitterness reducing effect of proline-specific proteases as such are also envisaged. For example, the incorporation of the endopeptidase in proteinaceous food products involving a fermentation step such as in cheeses or yogurt to suppress bitterness which may evolve on aging. Also in proteinaceous food products requiring treatment with proteases such as the production of enzyme modified cheeses or the production of protein hydrolysates for the flavour industry, the incorporation of the enzyme according to the invention will help to suppress bitterness.

Thus, the invention provides a method for the preparation of a food or feed product or a beverage which method comprises which method comprises incorporating a polypeptide or a composition of the invention during preparation of the food product, feed product or beverage. The invention also provides the use of a polypeptide or a composition of the invention in the preparation of a food or feed product or a beverage. Also provided by the invention is a food or feed product or beverage obtainable by the above method or use.

Applications for the polypeptides and compositions of the claims, for example in a food or a beverage are not limited to situations where suppression of a bitter taste is desired.

For example, a polypeptide of the invention may be contacted with a food protein to reduce its allergenicity. Several food proteins contain highly allergenic subfractions, such as wheat gluten that contains prolamines with proline-rich peptide sequences. These proteins can be subjected to the new enzyme to alleviate their antigenicity.

Accordingly, the invention provides a method for the preparation of a food or feed product or beverage which method comprises incorporating a polypeptide or a composition of the invention during preparation of the food product, feed product or beverage and wherein the resulting food or feed product substantially does not comprise celiac related epitopes.

In addition, the invention provides a polypeptide of the invention for use in the treatment or prophylaxis of a psychiatric disorder or a celiac disease linked disorder.

Proline rich dietary proteins such as caseins in bovine milk or glutens in cereals are known to resist proteolytic degradation in the human gastrointestinal tract. As a result proline rich peptides can build up and may lead to undesirable effects in specific groups of individuals. Some of these effects have been ascribed to the fact that the proline rich peptides act as opioids that bind to receptors in peripheral tissues and the central nervous system. For example, syndromes shown by autistic and schizophrenic patients have been linked with the consumption of proline rich dietary proteins. Other effects are the result of intolerance to proline rich peptides. For example specific proline rich sequences are responsible for the observed toxicity of gluten in celiac disease. Celiac disease is a widely prevalent autoimmune disease of the small intestine which can only be treated by a life-long gluten free diet. Celiac disease is occasionally also accompanied by psychiatric and neurological symptoms illustrating the far-reaching consequences a disturbed metabolism of proline rich peptides may have.

Use of a proline-specific protease to remove toxic proline rich peptides from food prior to consumption or for the oral supply of corrective enzymes to compensate for an inadequate intestinal digestion process is described in detail in WO 2005/027953

The polypeptide of the invention may thus be used to hydrolyse, for example at pH of below 5.5, proline rich peptides which are brought in relation with psychiatric disorders including autism, schizophrenia, ADHD, bipolar mood disorder and depression and celiac disease linked disorders like autoimmune disorders, especially type 1 diabetes, dermatitis herpetiformis, autoimmune thyroiditis, collagen diseases, autoimmune alopecia and autoimmune hepatitis and IBS. Accordingly, the invention provides a polypeptide according to the invention for use in the treatment or prophylaxis of a psychiatric disorder or a celiac disease linked disorder.

Advantageously the enzyme encoded by a polypeptide of the invention is used to produce food, for example beer or bread which is lowered in, for example substantially free from celiac related epitopes, preferably gluten epitopes, more preferably wheat or barley epitopes.

The incorporation of a polypeptide of the invention into any kind of dough may be desirable since it has been observed that this retards the staling of the products, such as breads, thereby obtained.

A proline-specific protease of the invention may be used in a method for generating proline-rich peptides. Such proline-rich peptides are desirable additions to various food or nutraceutical products as they have been implicated in anorectic action, in fibrinolytic and antithrombotic and antihypertensive effects, in protection of the gastric mucosa as well as the prevention of rheumatoid arthritis.

A further application is the addition of a polypeptide of the invention in animal feed to enhance protein utilisation. For example, addition of a polypeptide of the invention may be used to improve digestibility of hard-to-digest proline rich sequences present in the feed protein as well as to improved conversion rates of cheaply available vegetable proteins containing high levels of polyphenols.

In addition, the invention provides use of a polypeptide of the invention in the preparation of a beverage, for example in beer brewing. Barley proteins, for example, are rich in proline rich sequences and in their non-malted form cereal proteins are extremely difficult to degrade into the free amino acids required to create a suitable fermentable wort. Accordingly, incorporation of a polypeptide of the invention into the mashing process may be used to stimulate amino acid release from milled but non-malted barley so that a much richer wort is obtained. In a similar way beer fermentation from mashes containing a high proportion of other cheap and locally available cereals such as for example sorghum can be improved.

The invention also provides a method for the prevention or reduction of haze in a beverage. Such a method comprises adding a polypeptide of the invention to a beverage, or incorporating a polypeptide of the invention during preparation of the beverage thereby to prevent or reduce haze in the beverage. Herein, the term "beverage" includes any beverage at any stage of its preparation. Thus, a beverage is not only a beverage ready for consumption but also any composition used to prepare the beverage. For example, wort as used in beer preparation is encompassed by the term "beverage" as used herein. Also, the addition of a polypeptide of the invention during the preparation of a beverage to compositions that are not or not entirely liquid is intended to fall within the method according to the invention. A polypeptide of the invention added to a mash at the start of beer brewing is an example of such a composition. The use of proline-specific proteases in the reduction or prevention of haze in beer is described in detail in WO 02/46381 and WO 2007/101888.

Herein, the words peptide and protein are used interchangeably. In this text, the words "haze", "cloudiness" and "turbidity" are also used interchangeably. To quantify the amount of haze in a beverage, a turbidimeter is often used. In a turbidimeter the amount of light is measured that is scattered at a pre-described angle relative to the direction of the incident light beam. Turbidity measurements are very suitable for the measurement of haze formed as the result of protein-polyphenol interactions. The way in which turbidity measurements may be carried out in beverages to which a polypeptide of the invention has been added is described in detail in WO 2007/101888. When a proline-specific protease is used in a method to reduce or prevent haze, it appears that haze measurements may preferably be carried out using a 25 degree scatter angle.

A polyphenol is defined as a compound having a chemical structure which structure contains at least two aromatic rings substituted with at least one hydroxyl group or having a chemical structure which contains at least one aromatic ring substituted with at least two hydroxyl groups. Examples of polyphenols are tannins and flavonoids, which include for example catechins, flavonols and anthocyanins.

The method according to the invention may advantageously be applied to beer, wine and fruit juice. It may also advantageously be applied to alcoholic beverages other than beer and wine.

The term "beer" as used herein is intended to cover at least beer prepared from mashes prepared from unmalted cereals as well as all mashes prepared from malted cereals, and all mashes prepared from a mixture of malted and unmalted cereals. The term "beer" also covers beers prepared with adjuncts, and beers with all possible alcohol contents.

Fruit juice may be a juice obtained from for example red berries, strawberries, apples, pears, tomatoes, citrus fruits, vegetables etc.

The amount of a polypeptide of the invention that is added to a beverage in the method according to the invention may vary between wide limits. In a preferred embodiment of the method according to the invention at least 150 milli-units of proline-specific protease activity, whereby the activity is determined by an activity measurements using Z-Gly-Pro-pNA as a substrate, per gram protein in the beverage is added. More preferably, at least 500 milli-units of proline-specific protease is added to the beverage, and most preferably, at least 1 unit of proline-specific protease is added.

A maximum amount of a polypeptide of the invention to be added cannot be specified. The maximum amount is for example dependent on the desired amount of haze reduction or prevention, the composition of the beverage, the pH of the beverage and the pH at which the protease has its maximum activity.

A polypeptide of the invention may be added at different stages during the preparation of a beverage. During the preparation process of beer, a polypeptide of the invention may advantageously be added to a mash. The polypeptide of the invention may be added to a fermented beer before haze is formed. However, it is also possible that a polypeptide of the invention is added to a fermented beer after haze has been formed. A polypeptide of the invention may advantageously be added to the mashing or maturation step in a process for the preparation of beer.

During the preparation of a wine, a polypeptide of the invention may preferably added to a fermented wine. The polypeptide may advantageously be added after alcoholic fermentation or after malolactic fermentation in a process for the production of a wine.

In a process for the preparation of a fruit juice, a polypeptide of the invention may preferably be added during maceration or depectinization.

Since haze formation often occurs in acidic beverages such as for example beer, wine and fruit juice, a polypeptide of the invention having a prolyl specific activity at a pH value below 7 are preferably used. Most preferably, prolyl-specific proteases having a maximum prolyl specific activity at a pH value below 7 are used in the method according to the invention.

As is typical for enzyme activities, the activity of prolyl-specific proteases is dependent on the pH. In a preferred embodiment of the method according to the invention, a protease is added to the beverage having a maximum prolyl specific activity at a pH which corresponds to the pH of the beverage it is added to. Preferred beverages are protein containing beverages. In another preferred embodiment, the beverage contains proteins and polyphenols. Preferred beverages are beverages having a pH value below 7.

A proline-specific protease may comprise at least about 5 units per gram protein of the enzyme to be used, preferably about 10 units/g, more preferably about 25 units/g and even more preferably about 50 units/g.

According to one embodiment of the invention, the use of a polypeptide of the invention may allow the time needed for stabilisation of a beverage such as beer to be reduced (as compared to a process in which a proline-specific protease is not used). For example, the stabilisation period can be shortened to less than about 7 days. Preferably it is less than 6, 5, 4 or 3 days. More preferably, it is shortened to less than 2 or 1 days.

Most preferably, the stabilisation phase may be shortened to such an extent that on production scale, the duration of the stabilisation is reduced to the equivalent of the period required to cool the beer from the maturation phase to the desired temperature, for example the temperature desired for filtration and/or packaging of the beer. This period is conventionally called the cooling period. To shorten the stabilisation period to the time needed to cool the beer down to the desired temperature, is highly advantageous since then the duration of the stabilisation phase only depends on the cooling capacity available.

The beer from the maturation phase is traditionally cooled to a temperature from about -2°C up to and including about 0°C. However, use of a proline-specific protease according to the invention allows the stabilisation phase not only be shortened, but also to be performed at a higher temperature than conventionally used. Traditionally, there are stabilisation processes that are carried out at higher temperatures, but these processes take much more time, for example 6 weeks and more. Therefore, in another embodiment of the invention, the stabilisation period, which may typically be a shortened stabilisation period as defined above, is performed on a beer preferably cooled to at most about 2°C, more preferably to at most about 3°C, 4°C, 5°C or 6°C or even more preferably to at most about 7°C or about 8°C and most preferably to the desired temperature used for packaging, ie. for bottle or keg filling.

The desired temperature for packaging can differ from process to process, but is preferably carried out at a temperature up to and including 8°C, preferably around 7°C, in order to obtain the desired level of dissolved carbon dioxide in the beer. In a process where the beer is only cooled down to the temperature required for packaging, considerably less energy is required than in the processes known in the art.

In a preferred embodiment according to the invention, the stabilisation phase may be reduced to the period required to cool the beer from the temperature at the end of the maturation phase to the temperature desired for packaging, thereby omitting the conventional cold stabilisation period.

According to the invention, therefore, beer may be cooled to at most about 2°C, more preferably to at most about 3°C, 4°C, 5°C or 6°C, even more preferably to at most about 7°Cor 8°C and most preferably to the desired temperature used for packaging. The beer may then be packaged directly, i.e. in this embodiment, the beer is not held for any period of time at the temperature to which it has been cooled.

In the invention, further processing steps may be carried out to achieve additional clarification and/or stability if required. Indeed, if a shortened stabilisation phase is used which corresponds to the period required to cool the beer to the temperature desired for packaging, it may be desirable to treat the beer with PVPP and/or silica gel for example. This may help to ensure extended shelf-life stability.

The following Examples illustrate the invention:

### EXAMPLES

### Materials & Methods

Chromogenic peptides: all synthetic peptides were provided by Pepscan Presto (Almere, The Netherlands).

Spectrophotometric method for determining the prolyl-specific protease activity: The substrate solution is a 2 mM solution of N-carbobenzoxy-glycine-proline-p-nitro anilide (Z-Gly-Pro-pNA; m.w. 426.43; Bachem) made in a 0.1 M citric acid / 0.2 M disodium phosphate buffer pH 5.0 containing 40 % dioxan. To 1 mL of buffer pH 5.0, 250 µl of the substrate solution is added followed by 100 µl of the enzyme solution (larger or smaller volume amounts of enzyme solution should be compensated for by buffer solution). The reaction mixture is incubated at 37°C and the release of pNA is followed by measuring the absorbance increase at 410 nm.
Activity definition: 1 unit of proline specific protease is the enzyme activity that liberates 1µmol pNA from Z-Gly-Pro-pNA in 1 minute under described reaction conditions. In order to calculate concentrations a molar extinction coefficient (E) of 8,800 M⁻¹ is used.
SDS-PAGE: all materials used for SDS-PAGE and staining were purchased from Invitrogen (Carlsbad, CA, US). Samples were prepared using SDS buffer according to manufacturer's instructions and separated on 12% Bis-Tris gels using MES-SDS buffer system according to manufacturer's instructions. Staining was performed using Simply Blue Safe Stain (Collodial Coomassie G250).

### Example 1

### Cloning and expression of the proline-specific protease gene ZFX

*Penicillium chrysogenum* strain CBS 455.95 was grown for 3 days at 30 degrees Celsius in PDB (Potato dextrose broth, Difco) and chromosomal DNA was isolated from the mycelium using the Q-Biogene kit (catalog nr. 6540-600; Omnilabo International BV, Breda, the Netherlands), using the instructions of the supplier. This chromosomal DNA was used for the amplification of the coding sequence of the proline-specific protease gene using PCR.

To specifically amplify the proline-specific protease gene ZFX from the chromosomal DNA of *Penicillium chrysogenum* strain CBS 455.95, two PCR primers were designed. Primer sequences were partly obtained from a sequence that was found in the genomic DNA of *Penicillium chrysogenum* CBS 455.95 and is depicted in SEQ ID NO: 1. We found that this sequence has <50% identity with proline-specific protease gene sequences of *Aspergillus niger.* We describe here for the first time the efficient expression and characterization of a secreted *Penicillium* proline-specific protease. The protein sequence of the complete proline-specific protease protein, including potential pre- and pro-sequences is depicted in SEQ ID NO: 3.
ZFX-dir 5'-CACTTAATTAACTCATAGGCATCATGCATTTCTCAACTGTGGTTAAG (SEQ ID NO: 4)
ZFX-rev 5'-TTAGGCGCGCCTCGCAAGCTGATACGAAAGGG (SEQ ID NO: 5)

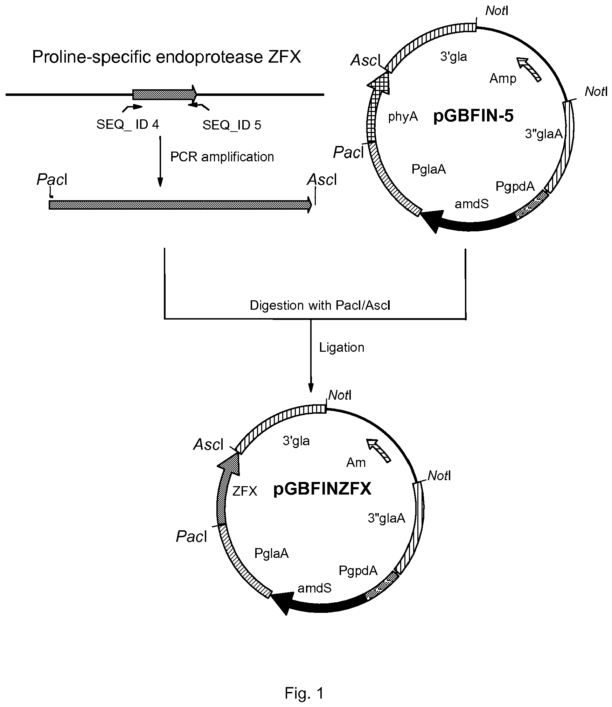

The first, direct PCR primer (ZFX-dir) contains 24 nucleotides ZJW coding sequence starting at the ATG start codon, preceded by a 23 nucleotides sequence including a *Pac*I restriction site (SEQ ID NO: 4). The second, reverse primer (ZFX-rev) contains nucleotides of the reverse complement strand of the region downstream of the ZFX coding sequence preceded by an *Asc*I restriction site (SEQ ID NO: 5). Using these primers we were able to amplify a 1.9 kb sized fragment with chromosomal DNA from *Penicillium chrysogenum* strain CBS 455.95 as template. The thus obtained 1.9 kb sized fragment was isolated, digested with *Pac*I and *Ascl* and purified. The *Pac*I / *Asc*I fragment comprising the ZFX coding sequences was exchanged with the *Pac*I / *Asc*I *phy*A fragment from expression vector pGBFIN-5 (WO 99/32617). Resulting plasmid is the ZFX expression vector named pGBFINZFX (see Figure 1), where the ZFX gene is functionally coupled to the glaA promoter of *Aspergillus niger.* The expression vector pGBFINZFX was linearized by digestion with *Not*I*,* which removes all *E. coli* derived sequences from the expression vector. The digested DNA was purified using phenol: chloroform: isoamylalcohol (24:23:1) extraction and precipitation with ethanol. These vectors were used to transform *Aspergillus niger* CBS513.88. An *Aspergillus niger* transformation procedure is extensively described in WO 98/46772. It is also described how to select for transformants on selective medium agar plates containing acetamide as sole nitrogen source, and to select targeted multicopy integrants. Preferably, *A. niger* transformants containing multiple copies of the expression cassette are selected for further generation of sample material. For the pGBFINZFX expression vector 30 *A. niger* transformants were purified; first by plating individual transformants on selective medium plates followed by plating a single colony on PDA (potato dextrose agar: PDB + 1.5% agar) plates. Spores of individual transformants were collected after growth for 1 week at 30 degrees Celsius. Spores were stored refrigerated and were used for the inoculation of liquid media.

The *A. niger* transformant strains were used for generation of sample material by cultivation of the strains in shake flask cultures. A useful method for cultivation of *A. niger* strains and separation of the mycelium from the culture broth is described in WO 98/46772. Cultivation medium was in CSM-MES (150 g maltose, 60 g Soytone (Difco), 15 g (NH₄)₂SO₄, 1 g NaH₂PO₄·H₂O, 1 g MgSO₄·7H₂O, 1 g L-arginine, 80 mg Tween-80, 20 g MES pH6.2 per liter medium). 5 ml samples were taken from duplicate cultivations on day 4-8 of the fermentation, centrifuged for 10 min at 5000 rpm in a Hereaus labofuge RF and supernatants were stored at -20°C until further analyses.

It became clear that the transformants containing the pGBFINZFX vector had a surprisingly efficient secretion of a protein of apparent molecular weight of approximately 65 kDa when analyzed with SDS-PAGE. Since this is almost identical to the molecular weight that is predicted from the protein sequence of ZFX, we presume that after removal of the signal sequence almost no glycosylation takes place when *Penicillium chrysogenum* proline-specific protease ZFX is secreted from *Aspergillus niger.*

Selected strains can be used for isolation and purification of a larger amount of *Penicillium* proline-specific protease, when fermentation and down-stream processing is scaled up. This enzyme can than be used for further analysis, and for the use in diverse industrial applications.

### Example 2

### The enzyme encoded by gene ZFX is a proline-specific protease

The fermentation broth of a selected *A. niger* transformant was recovered after which the liquid was filtered and then sterile filtered using a 0.22 micrometer Millipore filter. The clear liquid was then concentrated using a Pellicon ultrafiltration unit (Millipore, Bedford, MA, USA). To purify the enzyme activity encoded by gene ZFX, the concentrated liquid was then subjected to anion exchange chromatography using Q-sepharose FF XK (GE Healthcare Bio-Sciences, Diegem, Belgium). To that end, the concentrated liquid was equilibrated with 20 mM sodium citrate, pH 6.4 and applied to the column equilibrated in the same buffer. Column elution took place in twenty column volumes using a linear gradient from 0 to 1 mol/I NaCl in the application buffer. Eluted fractions were subjected to SDS-PAGE and stained (see Materials & Methods section). Those fractions showing a protein band corresponding with a molecular weight of approximately 65 kDa were pooled, dialized and again subjected to the same chromatographic and pooling procedure. The resulting preparation contained only a single protein band as judged by SDS-PAGE and staining.

To confirm the proteolytic activity of the purified protein, incubations were carried out with a full set of synthetic chromogenic peptides of the formula Z-Ala-Ala-X-pNA (Z: benzyloxycarbonyl; pNA: para-nitroanilide; X: any of all 20 amino acid residues). The individual substrates were first dissolved in DMSO and then diluted to the desired concentration of approximately 3 millimol/I in either 0.1 mol/l sodium acetate pH 4.0 or in 0.1 mol/I tris-HCl buffer pH 7.0. After addition of the enzyme, the activity assay was performed in a microtiterplate (MTP) using a Tecan Genios MTP reader running with Magellan software (Tecan, Salzburg, Vienna).

According to the results obtained, only peptide Z-Ala-Ala-Pro-pNA was efficiently cleaved demonstrating that the Penicillium derived ZFX enzyme as overexpressed in *A. niger* is indeed a proline-specific protease. Additional characterization of the enzyme using peptide Z-Ala-Ala-Pro-pNA dissolved in either Na-citrate-MES, MES-NaOH and Tris-HCl buffers revealed that, after 60 minutes of incubation at 37 degrees C, optimal proteolytic activity was deployed around pH 4.5.

### Example 3

### Proline-specific protease ZFX has a temperature optimum around 37°C

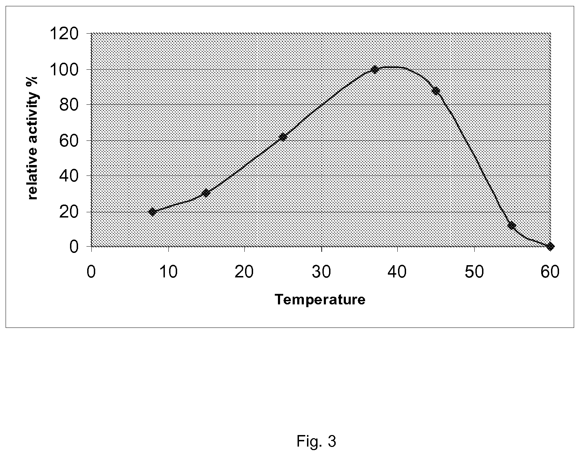

In order to test the temperature optimum of the proline-specific protease from *Penicillium,* the overexpressed ZFX enzyme, chromatographically purified as described in Example 2, was again incubated with peptide Z-Ala-Ala-Pro-pNA. A reaction mixture containing 440 microliter 0.1mol/l Na-citrate pH 5.0, 50 microliter of the chromogenic peptide in a concentration of 15 millimol/l and 10 microliter of the purified enzyme solution, was incubated for 70 minutes at different temperatures. Then the reaction was stopped by adding 0.5 ml 2% (w/w) Tris solution and the optical density at 405 nm was measured to quantify the cumulative enzymatic activity under these circumstances. The results obtained (see Figure 3) clearly demonstrate a temperature optimum of the proteolytic activity around 37°C.

### Example 4

### Enzyme ZFX is completely inactivated upon pasteurization

One of the industrial applications of the proline-specific protease from *A. niger* is its use in preventing chill haze formation in beer. In this application, enzyme activity should be lost during beer pasteurization. In view of the relatively low temperature optimum of the ZFX enzyme, we tested whether or not the enzyme ZFX would survive typical beer pasteurization conditions.

Rather than using beer, "reconstituted" beer was used for this test. The reason for this was our observation that the color change resulting from successful cleavage of the Pro-pNA peptide bond is blurred by the color present in beer. Therefore, the ZFX enzyme was dissolved in an industrially relevant dosage in a 50 millimol/I Na-acetate pH 4.5 buffer containing 5 % of ethanol. After subjecting this solution to the usual beer pasteurization treatment of 20 minutes at 60°C, the liquid was cooled to 37°C. Then the Z-Ala-Ala-Pro-pNA peptide.was added to an end concentration of 3 millimol/I to detect any residual proline-specific proteolytic activity. Even after prolonged incubation times no increase in OD 405 could be recorded illustrating that the Penicillium-derived ZFX enzyme was completely inactivated by the pasteurization treatment that was applied.

## Claims

1. A polypeptide having proline-specific protease activity which comprises the amino acid sequence set out in SEQ ID NO: 3 or an amino acid sequence encoded by the nucleotide sequence of SEQ ID NO: 1 and/or SEQ ID NO: 2, which has at least the same level of proline-specific protease activity as the polypeptide of SEQ ID NO: 3.

2. A polypeptide having proline-specific protease activity , which has at least 60%, 65% 70% 75%, 80%, 85%, 90%, 95%, 98% or 99% sequence identity with the sequence set out in SEQ ID NO: 3, which has at least the same level of proline-specific protease activity as the polypeptide of SEQ ID NO: 3.

3. A polynucleotide encoding a polypeptide having proline-specific protease activity which comprises:
(a) the nucleotide sequence as set out in SEQ ID NO: 1 and/or SEQ ID NO: 2;
(b) a nucleotide sequence which hybridizes under high stringency conditions with a polynucleotide being the reverse complement of SEQ ID NO: 1 and/or SEQ ID NO: 2;
(c) a nucleotide sequence having at least about 90% sequence identity with the nucleotide sequence of SEQ ID NO: 1 and/or SEQ ID NO: 2.

4. A polynucleotide which encodes a polypeptide according to claims 1 or 2.

5. A polynucleotide according to claims 3 or 4 which is a DNA sequence.

6. A polypeptide according to claims 1 or 2 or a polynucleotide according to any one of claims 3 or 5 obtainable from a fungus.

7. A polypeptide or polynucleotide according to claim 6 obtainable from *Penicillium chrysogenum.*

8. A vector incorporating a polynucleotide sequence according to any one of claims 3 to 7.

9. A vector according to claim 8 wherein the vector is an expression vector, for example wherein the polynucleotide sequence is operably linked with a regulatory sequence, allowing for expression of the polynucleotide sequence in a cell.

10. A vector according to claim 9 wherein the cell is a filamentous fungus cell.

11. A cell comprising a polypeptide according to any one of claims 1, 2, 6 or 7, a polynucleotide according to any one of claims 3 to 7 or a vector according to any one of claims 8 to 10.

12. A method for the preparation of a polypeptide having proline-specific protease activity, which method comprises cultivating a cell according to claim 11 under conditions which allow for expression of said polypeptide and, optionally, recovering the expressed polypeptide.

13. A composition comprising: (i) a polypeptide according to any one of claims 1, 2, 6, or 7; and, optionally, (ii) a protease which is different from the polypeptide in (i).

14. A method for the preparation of a protein hydrolysate, which method comprises contacting a protein substrate with a polypeptide according to any one of claims 1, 2, 6, or 7 or a composition according to claim 13.

15. Use of a polypeptide according to any one of claims 1, 2, 6, or 7 or a composition according to claim 13 in the preparation of a protein hydrolysate.

16. A method for the preparation of a food or feed product or a beverage which method comprises incorporating a polypeptide according to any one of claims 1, 2, 6, or 7, or a composition according to claim 13 during preparation of the food product, feed product or beverage.

17. A method according to claim 16, wherein the method is for the prevention or reduction of haze in a beverage.

18. A method according to claim 16 or 17, wherein the resulting food or feed product substantially does not comprise celiac related epitopes.

19. Use of a polypeptide according to any one of claims 1, 2, 6, or 7 or a composition according to claim 13, in the preparation of a food or feed product or a beverage.

## Patentansprüche

1. Polypeptid mit prolinspezifischer Proteaseaktivität, das die Aminosäuresequenz gemäß SEQ ID NO: 3 oder eine Aminosäuresequenz, die von der Nukleotidsequenz von SEQ ID NO: 1 und/oder SEQ ID NO: 2 codiert wird und die zumindest das gleiche Ausmaß an prolinspezifischer Proteaseaktivität wie das Polypeptid von SEQ ID NO: 3 aufweist, umfasst.

2. Polypeptid mit prolinspezifischer Proteaseaktivität, das mindestens 60%, 65%, 70%, 75%, 80%, 85%, 90%, 95%, 98% oder 99% Sequenzidentität zu der Sequenz gemäß SEQ ID NO: 3 aufweist und das mindestens dasselbe Ausmaß an prolinspezifischer Proteaseaktivität wie das Polypeptid von SEQ ID NO: 3 aufweist.

3. Polynukleotid, das für ein Polypeptid mit prolinspezifischer Proteaseaktivität codiert und das Folgendes umfasst:
(a) die Nukleotidsequenz gemäß SEQ ID NO: 1 und/oder SEQ ID NO: 2;
(b) eine Nukleotidsequenz, die unter hochstringenten Bedingungen mit einem Polynukleotid, das das reverse Komplement von SEQ ID NO: 1 und/oder SEQ ID NO: 2 ist, hybridisiert;
(c) eine Nukleotidsequenz mit mindestens ungefähr 90% Sequenzidentität zu der Nukleotidsequenz von SEQ ID NO: 1 und/oder SEQ ID NO: 2.

4. Polynukleotid, das für ein Polypeptid nach den Ansprüchen 1 oder 2 codiert.

5. Polynukleotid nach den Ansprüchen 3 oder 4, bei dem es sich um eine DNA-Sequenz handelt.

6. Polypeptid nach den Ansprüchen 1 oder 2 oder Polynukleotid nach einem der Ansprüche 3 oder 5, das von einem Pilz erhältlich ist.

7. Polypeptid oder Polynukleotid nach Anspruch 6, das von *Penicillium chrysogenum* erhältlich ist.

8. Vektor, der eine Polynukleotidsequenz nach einem der Ansprüche 3 bis 7 beinhaltet.

9. Vektor nach Anspruch 8, wobei es sich bei dem Vektor um einen Expressionsvektor handelt, zum Beispiel wobei die Polynukleotidsequenz operativ mit einer Regulationssequenz verknüpft ist und so die Expression der Polynukleotidsequenz in einer Zelle ermöglicht.

10. Vektor nach Anspruch 9, wobei es sich bei der Zelle um eine Fadenpilzzelle handelt.

11. Zelle, die ein Polypeptid nach einem der Ansprüche 1, 2, 6 oder 7, ein Polynukleotid nach einem der Ansprüche 3 bis 7 oder einen Vektor nach einem der Ansprüche 8 bis 10 umfasst.

12. Verfahren zur Herstellung eines Polypeptids mit prolinspezifischer Proteaseaktivität, das umfasst:
Kultivieren einer Zelle nach Anspruch 11 unter Bedingungen, die die Expression des Polypeptids ermöglichen, und gegebenenfalls Gewinnen des exprimierten Polypeptids.

13. Zusammensetzung, umfassend: (i) ein Polypeptid nach einem der Ansprüche 1, 2, 6 oder 7; und gegebenenfalls (ii) eine Protease, die sich von dem Polypeptid in (i) unterscheidet.

14. Verfahren zur Herstellung eines Proteinhydrolysats, das beinhaltet: Inkontaktbringen eines Proteinsubstrats mit einem Polypeptid nach einem der Ansprüche 1, 2, 6 oder 7 oder einer Zusammensetzung nach Anspruch 13.

15. Verwendung eines Polypeptids nach einem der Ansprüche 1, 2, 6 oder 7 oder eine Zusammensetzung nach Anspruch 13 in der Herstellung eines Proteinhydrolysats.

16. Verfahren zur Herstellung eines Nahrungs- oder Futtermittelprodukts oder eines Getränks, das umfasst:
Einarbeiten eines Polypeptids nach einem der Ansprüche 1, 2, 6 oder 7 oder einer Zusammensetzung nach Anspruch 13 während der Herstellung des Nahrungsmittelprodukts, Futtermittelprodukts oder Getränks.

17. Verfahren nach Anspruch 16, wobei das Verfahren der Vorbeugung oder Verminderung von Trübheit in einem Getränk dient.

18. Verfahren nach Anspruch 16 oder 17, wobei das entstandene Nahrungs- oder Futtermittelprodukt im Wesentlichen keine mit Zöliakie in Zusammenhang stehenden Epitope umfasst.

19. Verwendung eines Polypeptids nach einem der Ansprüche 1, 2, 6 oder 7 oder einer Zusammensetzung nach Anspruch 13 in der Herstellung eines Nahrungs- oder Futtermittelprodukts oder eines Getränks.

## Revendications

1. Polypeptide ayant une activité protéase spécifique de la proline et comprenant la séquence d'acides aminés présentée dans SEQ ID NO : 3 ou une séquence d'acides aminés encodée par la séquence nucléotidique de SEQ ID NO : 1 et/ou SEQ ID NO : 2, qui présente au moins le même niveau d'activité protéase spécifique de la proline que le polypeptide de SEQ ID NO : 3.

2. Polypeptide ayant une activité protéase spécifique de la proline, qui présente au moins 60, 65, 70, 75, 80, 85, 90, 95, 98 ou 99 % d'identité de séquence avec la séquence présentée dans SEQ ID NO : 3, et qui présente au moins le même niveau d'activité protéase spécifique de la proline que le polypeptide de SEQ ID NO : 3.

3. Polynucléotide, codant pour un polypeptide ayant une activité protéase spécifique de la proline, qui comprend :
(a) la séquence nucléotidique présentée dans SEQ ID NO : 1 et/ou SEQ ID NO : 2 ;
(b) une séquence nucléotidique qui s'hybride dans des conditions de stringence élevée avec un polynucléotide correspondant au complément inverse de SEQ ID NO : 1 et/ou SEQ ID NO : 2 ;
(c) une séquence nucléotidique présentant au moins environ 90 % d'identité de séquence avec la séquence nucléotidique de SEQ ID NO : 1 et/ou SEQ ID NO : 2.

4. Polynucléotide qui code pour un polypeptide selon les revendications 1 ou 2.

5. Polynucléotide selon les revendications 3 ou 4, qui est une séquence d'ADN.

6. Polypeptide selon les revendications 1 ou 2 ou polynucléotide selon l'une quelconque des revendications 3 ou 5 pouvant être obtenu à partir d'un champignon.

7. Polypeptide ou polynucléotide selon la revendication 6 pouvant être obtenu à partir de *Penicillium chrysogenum.*

8. Vecteur intégrant une séquence polynucléotidique selon l'une quelconque des revendications 3 à 7.

9. Vecteur selon la revendication 8, dans lequel le vecteur est un vecteur d'expression, par exemple dans lequel la séquence polynucléotidique est en liaison fonctionnelle avec une séquence régulatrice, ce qui permet l'expression de la séquence polynucléotidique dans une cellule.

10. Vecteur selon la revendication 9, dans lequel la cellule est une cellule fongique filamenteuse.

11. Cellule comprenant un polypeptide selon l'une quelconque des revendications 1, 2, 6 ou 7, polynucléotide selon l'une quelconque des revendications 3 à 7 ou vecteur selon l'une quelconque des revendications 8 à 10.

12. Procédé de préparation d'un polypeptide ayant une activité protéase spécifique de la proline, lequel procédé comprend la culture d'une cellule selon la revendication 11 dans des conditions qui permettent l'expression dudit polypeptide et, éventuellement, le recueil du polypeptide exprimé.

13. Composition comprenant : (i) un polypeptide selon l'une quelconque des revendications 1, 2, 6 ou 7 ; et, éventuellement, (ii) une protéase qui est différente du polypeptide de (i).

14. Procédé de préparation d'un hydrolysat de protéines, lequel procédé comprend la mise en contact d'un substrat protéique avec un polypeptide selon l'une quelconque des revendications 1, 2, 6 ou 7 ou avec une composition selon la revendication 13.

15. Utilisation d'un polypeptide selon l'une quelconque des revendications 1, 2, 6 ou 7 ou d'une composition selon la revendication 13 dans la préparation d'un hydrolysat de protéines.

16. Procédé de préparation d'un produit alimentaire destiné à la consommation humaine ou animale ou d'une boisson, lequel procédé comprend l'incorporation d'un polypeptide selon l'une quelconque des revendications 1, 2, 6 ou 7, ou d'une composition selon la revendication 13 durant la préparation du produit alimentaire destiné à la consommation humaine ou animale ou de la boisson.

17. Procédé selon la revendication 16, dans lequel le procédé sert à la prévention ou à la réduction de la turbidité d'une boisson.

18. Procédé selon les revendications 16 ou 17, dans lequel le produit alimentaire destiné à la consommation humaine ou animale résultant ne comprend essentiellement pas d'épitopes associés à la maladie coeliaque.

19. Utilisation d'un polypeptide selon l'une quelconque des revendications 1, 2, 6 ou 7 ou d'une composition selon la revendication 13, dans la préparation d'un produit alimentaire destiné à la consommation humaine ou animale ou d'une boisson.


### SEQUENCE LISTING

<110> DSM IP ASSETS B.V.
<120> PROLINE-SPECIFIC PROTEASE
<130> 26633-WO-PCT
<160> 5
<170> Patent In version 3.2
<210> 1
   <211> 2180
   <212> DNA
   <213> Penicillium chrysogenum
<400> 1
   cacaaacgca accatattgt tcttatcata ttgttctcgg ggtacattta ttaccccgat      60
   tttgcatttt cccagttgtt acctatctct gtaatgcata tatattccat aagagaccca     120
   gcagtgagat aatccaaatc tgttctatat ccgtgtatcc taggctgggg gtttgagaga     180
   ctgccaaggc actctcagta tgcatttctc aactgtggtt aaggggattg caaccctgac     240
   tgcgctgtcc ggtacagctg caggaattgt cacatcgcag ttcaagcggg atctgaagct     300
   ttccgctgag ctggggattc atccagatac cctgctcggt caaaagacca cggtgcatga     360
   cgttaccaac tcccagcttg atgagaccat tgaggcagag tatgtttcgg tatgtgtcta     420
   tgcttcccaa tgccgaagat ggtgataaat gctgacaatt tatagatccc aattgaccac     480
   agcaactctt cggttggcca ctatcggaac cgctattggg tatccgaaga gcattacaag     540
   gaggacggac cgatcttcgt gtttgatgtg ggcgaatcaa ccgcggagcc tgcggggcaa     600
   acctatctaa gcaattcgag cacgttcttt taccagttgg ttaaagagtt cggtggaatt     660
   ggtatcgttt gggagcaccg gtttgtgtcc cctccccagt gaaagcccgg gctcaactaa     720
   ccaaaaccag gtactatggg gattctcttc cctacaacgt cagcctggat atggagcctg     780
   aacatctcca gtatctcaac aataagcagg ctctggcaga cataccatac tttgcggccc     840
   aattcactcg ccaagattac agcgatgtgg acctgactcc agctggaaca ccatgggtca     900
   tggtcggagg ctcgtacgct ggcatgcgcg cggcgtttac ccgtcaatcg tatcccgata     960
   ctatttacgc tgctttcgct tcgtccgccc ccgtcgaagc acgaatcgac atgagtgtct    1020
   attttgacca ggtgtatgac ggcatggtta catatggaca cttgaactgc accagggaca    1080
   tcaaggccgc actggagtat atcgacgagc aactctccaa gagcgaatcg gccgcagcgg    1140
   ccatcaagcg ggaattcttc ggtgaaggcg ccgaaaagaa cagcaacgga gatttcaccg    1200
   ccgcactcgt gacgatttac aactttttcc agtcttacgg tatgggaggt ggtgtgggca   1260
   gcctggagtc attctgcgaa cacatggaga ccgacccgaa gactagcgca gcggcaccct  1320
   ctgaaggatt cgctccgacc cgggggaaga agtatgtcgc ggagcgatat gcgtcatggc   1380
   cggttttcac acaggtagtc aatatgaata tgaagaccaa ctgcaaaaag cttgaaacca   1440
   gcgaaccgct cgagtgcgat ctgggaccac catccgccaa tccagacacc atcagctgga   1500
   catggcagta ctgcactgaa tggggatact tccaaaccaa taacttcgga gctcactcgt   1560
   tgctgtcaaa ataccagacc ctcgaatatg cacaggaata ttgcaacaga ttcttcccgg   1620
   aggctgttgc gaagggtctg ttccccaaac acccgcagac cgaggcgacc aatgcggaga   1680
   ccggcggatg gtctattcgt ccgtccaacg tttactggag cggcggccag tttgatccgt   1740
   ggcggacttt gtctcccctc gcggagacac ggttaggccc acagggtgtg actctgacga   1800
   cagagatccc caagtgcaat gtggagacgg aggaaaatac gatcttcggc tacatcatgc   1860
   agaaggcgga gcactgcttt gatttccaca cggcctttgt acctggggcg atttctcggg   1920
   gatattttca aacggcattg aaagagtggc ttggatgttt caaagccaag actcgttaaa   1980
   ccttgacaaa tgttcaacgt tcaacggata tcgtgactca ttttgacttt ggattttctc   2040
   cttttgacac tattcccctt tcgtatcagc ttgcgatcct ccccgtcttt tctcacgtgg   2100
   agctctaccg aggccgtgga catatagcat tctgtgaatt aaaattccat ctcacatatt   2160
   taattgctcg ttttctttca                                               2180
<210> 2
   <211> 1674
   <212> DNA
   <213> Penicillium chrysogenum
<220>
   <221> CDS
   <222> (1)..(1671)
<400> 2
   atg cat ttc tca act gtg gtt aag ggg att gca acc ctg act gcg ctg      48
   Met His Phe Ser Thr Val Val Lys Gly Ile Ala Thr Leu Thr Ala Leu
   1               5                   10                  15
   tcc ggt aca gct gca gga att gtc aca tcg cag ttc aag cgg gat ctg      96
   Ser Gly Thr Ala Ala Gly Ile Val Thr Ser Gln Phe Lys Arg Asp Leu
               20                  25                  30
   aag ctt tcc gct gag ctg ggg att cat cca gat acc ctg ctc ggt caa     144
   Lys Leu Ser Ala Glu Leu Gly Ile His Pro Asp Thr Leu Leu Gly Gln
               35                  40                  45
   aag acc acg gtg cat gac gtt acc aac tcc cag ctt gat gag acc att       192
   Lys Thr Thr Val His Asp Val Thr Asn Ser Gln Leu Asp Glu Thr Ile
       50                  55                  60
   gag gca gag tat gtt tcg atc cca att gac cac agc aac tct tcg gtt       240
   Glu Ala Glu Tyr Val Ser Ile Pro Ile Asp His Ser Asn Ser Ser Val
   65                  70                  75                  80
   ggc cac tat cgg aac cgc tat tgg gta tcc gaa gag cat tac aag gag       288
   Gly His Tyr Arg Asn Arg Tyr Trp Val Ser Glu Glu His Tyr Lys Glu
                   85                  90                  95
   gac gga ccg atc ttc gtg ttt gat gtg ggc gaa tca acc gcg gag cct       336
   Asp Gly Pro Ile Phe Val Phe Asp Val Gly Glu Ser Thr Ala Glu Pro
               100                 105                 110
   gcg ggg caa acc tat cta agc aat tcg agc acg ttc ttt tac cag ttg       384
   Ala Gly Gln Thr Tyr Leu Ser Asn Ser Ser Thr Phe Phe Tyr Gln Leu
           115                 120                 125
   gtt aaa gag ttc ggt gga att ggt atc gtt tgg gag cac cgg tac tat       432
   Val Lys Glu Phe Gly Gly Ile Gly Ile Val Trp Glu His Arg Tyr Tyr
       130                 135                 140
   ggg gat tct ctt ccc tac aac gtc agc ctg gat atg gag cct gaa cat       480
   Gly Asp Ser Leu Pro Tyr Asn Val Ser Leu Asp Met Glu Pro Glu His
   145                 150                 155                 160
   ctc cag tat ctc aac aat aag cag gct ctg gca gac ata cca tac ttt       528
   Leu Gln Tyr Leu Asn Asn Lys Gln Ala Leu Ala Asp Ile Pro Tyr Phe
                   165                 170                 175
   gcg gcc caa ttc act cgc caa gat tac agc gat gtg gac ctg act cca       576
   Ala Ala Gln Phe Thr Arg Gln Asp Tyr Ser Asp Val Asp Leu Thr Pro
               180                 185                 190
   gct gga aca cca tgg gtc atg gtc gga ggc tcg tac gct ggc atg cgc       624
   Ala Gly Thr Pro Trp Val Met Val Gly Gly Ser Tyr Ala Gly Met Arg
           195                 200                 205
   gcg gcg ttt acc cgt caa tcg tat ccc gat act att tac gct gct ttc       672
   Ala Ala Phe Thr Arg Gln Ser Tyr Pro Asp Thr Ile Tyr Ala Ala Phe
       210                 215                 220
   gct tcg tcc gcc ccc gtc gaa gca cga atc gac atg agt gtc tat ttt       720
   Ala Ser Ser Ala Pro Val Glu Ala Arg Ile Asp Met Ser Val Tyr Phe
   225                 230                 235                 240
   gac cag gtg tat gac ggc atg gtt aca tat gga cac ttg aac tgc acc       768
   Asp Gln Val Tyr Asp Gly Met Val Thr Tyr Gly His Leu Asn Cys Thr
                   245                 250                 255
   agg gac atc aag gcc gca ctg gag tat atc gac gag caa ctc tcc aag       816
   Arg Asp Ile Lys Ala Ala Leu Glu Tyr Ile Asp Glu Gln Leu Ser Lys
               260                 265                 270
   agc gaa tcg gcc gca gcg gcc atc aag cgg gaa ttc ttc ggt gaa ggc      864
   Ser Glu Ser Ala Ala Ala Ala Ile Lys Arg Glu Phe Phe Gly Glu Gly
           275                 280                 285
   gcc gaa aag aac agc aac gga gat ttc acc gcc gca ctc gtg acg att      912
   Ala Glu Lys Asn Ser Asn Gly Asp Phe Thr Ala Ala Leu Val Thr Ile
       290                 295                 300
   tac aac ttt ttc cag tct tac ggt atg gga ggt ggt gtg ggc agc ctg      960
   Tyr Asn Phe Phe Gln Ser Tyr Gly Met Gly Gly Gly Val Gly Ser Leu
   305                 310                 315                 320
   gag tca ttc tgc gaa cac atg gag acc gac ccg aag act agc gca gcg     1008
   Glu Ser Phe Cys Glu His Met Glu Thr Asp Pro Lys Thr Ser Ala Ala
                   325                 330                 335
   gca ccc tct gaa gga ttc gct ccg acc cgg ggg aag aag tat gtc gcg     1056
   Ala Pro Ser Glu Gly Phe Ala Pro Thr Arg Gly Lys Lys Tyr Val Ala
               340                 345                 350
   gag cga tat gcg tca tgg ccg gtt ttc aca cag gta gtc aat atg aat     1104
   Glu Arg Tyr Ala Ser Trp Pro Val Phe Thr Gln Val Val Asn Met Asn
           355                 360                 365
   atg aag acc aac tgc aaa aag ctt gaa acc agc gaa ccg ctc gag tgc     1152
   Met Lys Thr Asn Cys Lys Lys Leu Glu Thr Ser Glu Pro Leu Glu Cys
       370                 375                 380
   gat ctg gga cca cca tcc gcc aat cca gac acc atc agc tgg aca tgg     1200
   Asp Leu Gly Pro Pro Ser Ala Asn Pro Asp Thr Ile Ser Trp Thr Trp
   385                 390                 395                 400
   cag tac tgc act gaa tgg gga tac ttc caa acc aat aac ttc gga gct     1248
   Gln Tyr Cys Thr Glu Trp Gly Tyr Phe Gln Thr Asn Asn Phe Gly Ala
                   405                 410                 415
   cac tcg ttg ctg tca aaa tac cag acc ctc gaa tat gca cag gaa tat     1296
   His Ser Leu Leu Ser Lys Tyr Gln Thr Leu Glu Tyr Ala Gln Glu Tyr
               420                 425                 430
   tgc aac aga ttc ttc ccg gag gct gtt gcg aag ggt ctg ttc ccc aaa     1344
   Cys Asn Arg Phe Phe Pro Glu Ala Val Ala Lys Gly Leu Phe Pro Lys
           435                 440                 445
   cac ccg cag acc gag gcg acc aat gcg gag acc ggc gga tgg tct att     1392
   His Pro Gln Thr Glu Ala Thr Asn Ala Glu Thr Gly Gly Trp Ser Ile
       450                 455                 460
   cgt ccg tcc aac gtt tac tgg agc ggc ggc cag ttt gat ccg tgg cgg     1440
   Arg Pro Ser Asn Val Tyr Trp Ser Gly Gly Gln Phe Asp Pro Trp Arg
   465                 470                 475                 480
   act ttg tct ccc ctc gcg gag aca cgg tta ggc cca cag ggt gtg act     1488
   Thr Leu Ser Pro Leu Ala Glu Thr Arg Leu Gly Pro Gln Gly Val Thr
                   485                 490                 495
   ctg acg aca gag atc ccc aag tgc aat gtg gag acg gag gaa aat acg     1536
   Leu Thr Thr Glu Ile Pro Lys Cys Asn Val Glu Thr Glu Glu Asn Thr
               500                 505                 510
   atc ttc ggc tac atc atg cag aag gcg gag cac tgc ttt gat ttc cac     1584
   Ile Phe Gly Tyr Ile Met Gln Lys Ala Glu His Cys Phe Asp Phe His
           515                 520                 525
   acg gcc ttt gta cct ggg gcg att tct cgg gga tat ttt caa acg gca     1632
   Thr Ala Phe Val Pro Gly Ala Ile Ser Arg Gly Tyr Phe Gln Thr Ala
       530                 535                 540
   ttg aaa gag tgg ctt gga tgt ttc aaa gcc aag act cgt taa             1674
   Leu Lys Glu Trp Leu Gly Cys Phe Lys Ala Lys Thr Arg
   545                 550                 555
<210> 3
   <211> 557
   <212> PRT
   <213> Penicillium chrysogenum
<400> 3
   Met His Phe Ser Thr Val Val Lys Gly Ile Ala Thr Leu Thr Ala Leu
   1               5                   10                  15
   Ser Gly Thr Ala Ala Gly Ile Val Thr Ser Gln Phe Lys Arg Asp Leu
               20                  25                  30
   Lys Leu Ser Ala Glu Leu Gly Ile His Pro Asp Thr Leu Leu Gly Gln
           35                  40                  45
   Lys Thr Thr Val His Asp Val Thr Asn Ser Gln Leu Asp Glu Thr Ile
       50                  55                  60
   Glu Ala Glu Tyr Val Ser Ile Pro Ile Asp His Ser Asn Ser Ser Val
   65                  70                  75                  80
   Gly His Tyr Arg Asn Arg Tyr Trp Val Ser Glu Glu His Tyr Lys Glu
                   85                  90                  95
   Asp Gly Pro Ile Phe Val Phe Asp Val Gly Glu Ser Thr Ala Glu Pro
               100                 105                 110
   Ala Gly Gln Thr Tyr Leu Ser Asn Ser Ser Thr Phe Phe Tyr Gln Leu
           115                 120                 125
   Val Lys Glu Phe Gly Gly Ile Gly Ile Val Trp Glu His Arg Tyr Tyr
   130 135 140
   Gly Asp Ser Leu Pro Tyr Asn Val Ser Leu Asp Met Glu Pro Glu His
   145 150 155 160
   Leu Gln Tyr Leu Asn Asn Lys Gln Ala Leu Ala Asp Ile Pro Tyr Phe
   165 170 175
   Ala Ala Gln Phe Thr Arg Gln Asp Tyr Ser Asp Val Asp Leu Thr Pro
   180 185 190
   Ala Gly Thr Pro Trp Val Met Val Gly Gly Ser Tyr Ala Gly Met Arg
   195 200 205
   Ala Ala Phe Thr Arg Gln Ser Tyr Pro Asp Thr Ile Tyr Ala Ala Phe
   210 215 220
   Ala Ser Ser Ala Pro Val Glu Ala Arg Ile Asp Met Ser Val Tyr Phe
   225 230 235 240
   Asp Gln Val Tyr Asp Gly Met Val Thr Tyr Gly His Leu Asn Cys Thr
   245 250 255
   Arg Asp Ile Lys Ala Ala Leu Glu Tyr Ile Asp Glu Gln Leu Ser Lys
   260 265 270
   Ser Glu Ser Ala Ala Ala Ala Ile Lys Arg Glu Phe Phe Gly Glu Gly
   275 280 285
   Ala Glu Lys Asn Ser Asn Gly Asp Phe Thr Ala Ala Leu Val Thr Ile
   290 295 300
   Tyr Asn Phe Phe Gln Ser Tyr Gly Met Gly Gly Gly Val Gly Ser Leu
   305 310 315 320
   Glu Ser Phe Cys Glu His Met Glu Thr Asp Pro Lys Thr Ser Ala Ala
   325 330 335
   Ala Pro Ser Glu Gly Phe Ala Pro Thr Arg Gly Lys Lys Tyr Val Ala
   340 345 350
   Glu Arg Tyr Ala Ser Trp Pro Val Phe Thr Gln Val Val Asn Met Asn
   355 360 365
   Met Lys Thr Asn Cys Lys Lys Leu Glu Thr Ser Glu Pro Leu Glu Cys
       370                 375                 380
   Asp Leu Gly Pro Pro Ser Ala Asn Pro Asp Thr Ile Ser Trp Thr Trp
   385                 390                 395                 400
   Gln Tyr Cys Thr Glu Trp Gly Tyr Phe Gln Thr Asn Asn Phe Gly Ala
                   405                 410                 415
   His Ser Leu Leu Ser Lys Tyr Gln Thr Leu Glu Tyr Ala Gln Glu Tyr
               420                 425                 430
   Cys Asn Arg Phe Phe Pro Glu Ala Val Ala Lys Gly Leu Phe Pro Lys
           435                 440                 445
   His Pro Gln Thr Glu Ala Thr Asn Ala Glu Thr Gly Gly Trp Ser Ile
       450                 455                 460
   Arg Pro Ser Asn Val Tyr Trp Ser Gly Gly Gln Phe Asp Pro Trp Arg
   465                 470                 475                 480
   Thr Leu Ser Pro Leu Ala Glu Thr Arg Leu Gly Pro Gln Gly Val Thr
                   485                 490                 495
   Leu Thr Thr Glu Ile Pro Lys Cys Asn Val Glu Thr Glu Glu Asn Thr
               500                 505                 510
   Ile Phe Gly Tyr Ile Met Gln Lys Ala Glu His Cys Phe Asp Phe His
           515                 520                 525
   Thr Ala Phe Val Pro Gly Ala Ile Ser Arg Gly Tyr Phe Gln Thr Ala
       530                 535                 540
   Leu Lys Glu Trp Leu Gly Cys Phe Lys Ala Lys Thr Arg
   545                 550                 555
<210> 4
   <211> 47
   <212> DNA
   <213> Artificial sequence
<220>
   <223> Oligonucleotide primer
<400> 4
   cacttaatta actcataggc atcatgcatt tctcaactgt ggttaag 47
<210> 5
   <211> 32
   <212> DNA
   <213> Artificial sequence
<220>
   <223> Oligonucleotide primer
<400> 5
   ttaggcgcgc ctcgcaagct gatacgaaag gg 32